# EUROPEAN PATENT APPLICATION

(11) **EP 4 545 533 A1**
(43) Date of publication of application: **30.04.2025**
(21) Application number: 23862358.1
(22) Date of filing: 05.09.2023
(51) Int. Cl.: C07D 487/10, A61K 31/4188, A61P 35/00

(54) **SPIRO COMPOUND AND USE**

(30) Priority: 07.09.2022 CN 202211085342
(71) Applicant: Hitgen Inc., Chengdu, Sichuan 610200 (CN)
(72) Inventor: LI, Jin, Chengdu, Sichuan 610200 (CN); LIU, Chuan, Chengdu, Sichuan 610200 (CN); XIA, Shuai, Chengdu, Sichuan 610200 (CN); DOU, Dengfeng, Chengdu, Sichuan 610200 (CN); SHEN, Jianbo, Chengdu, Sichuan 610200 (CN); XIANG, Sichuan, Chengdu, Sichuan 610200 (CN); CAI, Longying, Chengdu, Sichuan 610200 (CN); DONG, Xiaofei, Chengdu, Sichuan 610200 (CN)
(74) Representative: Vitina, Maruta
(86) International application number: PCT/CN2023/116880
(87) International publication number: WO 2024/051664

(57) **Abstract**

A compound of the following formula: 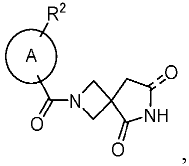 which is capable of binding to an E3 ubiquitin ligase cereblon (CRBN). An application of the compound in the preparation of drugs for the treatment of diseases associated with abnormal cell proliferation is also provided.

## Description

### TECHNICAL FIELD

This application relates to pharmaceuticals, and more particularly to a novel spiro ligand compound capable of binding to a cereblon E3 ubiquitin ligase (CRBN) and an application thereof.

### BACKGROUND

Protein degradation is a highly regulated process essential for maintaining cellular homeostasis. The selective recognition and removal of damaged, misfolded, or excess proteins are achieved through a ubiquitin-proteasome pathway (UPP). The UPP is responsible for clearing defective proteins, and is characterized by its ATP-dependency, high efficiency and high selectivity. The catalytic component of UPP is the E3 ubiquitin ligase, which first needs to recruit the to-be-degraded protein. PROTACs (proteolysis targeting chimeras) technology is designed based on the UPP, in which the target protein ligand is linked to the E3 ligase ligand with appropriate chemical bonds, enabling the recognition of the target protein and enhancing the binding affinity between the E3 ligase and the target protein. This results in targeted ubiquitination and degradation of the target protein, with excellent catalytic activity, high efficiency and high selectivity.

Multiple ubiquitin molecules mark proteins through the covalent linkage between the terminal lysine residues and the E3 ubiquitin ligase for proteasomal degradation. In this process, the proteins are digested into small peptides and eventually into their constituent amino acids, which are used for the synthesis of new proteins. Defective proteasomal degradation is associated with a variety of clinical diseases, including Alzheimer's disease, Parkinson's disease, Huntington's disease, muscular dystrophy, cardiovascular diseases, and cancer.

Cereblon, as a part of an E3 ubiquitin ligase complex, is a thalidomide-binding protein, which functions as a substrate receptor to selectively target the ubiquitinated proteins. Cereblon, encoded by the human *cereblon* (*CRBN*) gene, interacts with the damaged DNA-binding protein 1 (DDB1), Cullin-4A (CUL4A), and the Cullin-1 regulator (ROC1) to form the E3 ubiquitin ligase complex. This complex, which can ubiquitinate a series of proteins (the specific mechanism remains unknown). Cereblon is currently one of the commonly-used E3 ligases applied in the PROTACs technology.

The present application discloses a novel class of spiro compounds that can serve as effective CRBN ligands. These compounds can further be used to synthesize corresponding PROTACs bifunctional compounds, which can be applied to the treatment of various medical diseases, especially those diseases associated with abnormal cell proliferation.

### SUMMARY

In a first aspect, this application provides a compound of formula (I), or a stereoisomer, a deuterated compound or a pharmaceutically acceptable salt thereof: 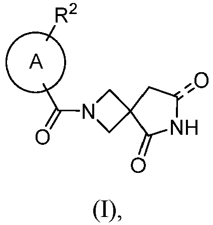 wherein:
==∘ represents presence or absence of oxygen substitution;
A ring is selected from the group consisting of 3 to 12-membered cycloalkyl, 4 to 12-membered heterocycloalkyl, 6 to 10-membered aromatic ring, and 5 to 10-membered heteroaromatic ring, wherein cycloalkyl, heterocycloalkyl, aromatic ring and heteroaromatic ring are independently unsubstituted or substituted with one, two, three or four R^{A1};
each R^{A1} is independently selected from the group consisting of hydrogen, halogen, cyano group, nitro, =O, =S, =CR^{A2}R^{A3}, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR^{A2}, -C₀₋₄ alkylidene-OC(O)R^{A2}, -C₀₋₄ alkylidene-SR^{A2}, -C₀₋₄ alkylidene-S(O)₂R^{A2}, -C₀₋₄ alkylidene-S(O)R^{A2}, -C₀₋₄ alkylidene-S(O)₂NR^{A2}R^{A3}, -C₀₋₄ alkylidene-S(O)NR^{A2}R^{A3}, -C₀₋₄ alkylidene-C(O)R^{A2}, -C₀₋₄ alkylidene-C(O)OR^{A2}, -C₀₋₄ alkylidene-C(O)NR^{A2}R^{A3}, -C₀₋₄ alkylidene-NR^{A2}R^{A3}, -C₀₋₄ alkylidene-NR^{A2}C(O)R^{A3}, -C₀₋₄ alkylidene-NR^{A2}S(O)₂R^{A3}, -C₀₋₄ alkylidene-NR^{A2}S(O)R^{A3}, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R^{A4};
each R^{A4} is independently selected from the group consisting of hydrogen, halogen, cyano group, nitro, =O, =S, =CR^{A2}R^{A3}, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR^{A2}, -C₀₋₄ alkylidene-OC(O)R^{A2}, -C₀₋₄ alkylidene-SR^{A2}, -C₀₋₄ alkylidene-S(O)₂R^{A2}, -C₀₋₄ alkylidene-S(O)R^{A2}, -C₀₋₄ alkylidene-S(O)₂NR^{A2}R^{A3}, -C₀₋₄ alkylidene-S(O)NR^{A2}R^{A3}, -C₀₋₄ alkylidene-C(O)R^{A2}, -C₀₋₄ alkylidene-C(O)OR^{A2}, -C₀₋₄ alkylidene-C(O)NR^{A2}R^{A3}, -C₀₋₄ alkylidene-NR^{A2}R^{A3}, -C₀₋₄ alkylidene-NR^{A2}C(O)R^{A3}, -C₀₋₄ alkylidene-NR^{A2}S(O)₂R^{A3} and -C₀₋₄ alkylidene-NR^{A2}S(O)R^{A3};
R^{A2} and R^{A3} are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl;
R² is selected from the group consisting of hydrogen, halogen, cyano group, nitro, =O, =S, =CR²¹R²², -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-SR²¹, -C₀₋₄ alkylidene-S(O)₂R²¹, -C₀₋₄ alkylidene-S(O)R²¹, -C₀₋₄ alkylidene-S(O)₂NR²¹R²², -C₀₋₄ alkylidene-S(O)NR²¹R²², -C₀₋₄ alkylidene-C(O)R²¹, -C₀₋₄ alkylidene-C(O)OR²¹, -C₀₋₄ alkylidene-C(O)NR²¹R²², -C₀₋₄ alkylidene-NR²¹R²², -C₀₋₄ alkylidene-NR²¹C(O)R²², -C₀₋₄ alkylidene-NR²¹S(O)₂R²², -C₀₋₄ alkylidene-NR²¹S(O)R²², -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²³;
each R²³ is independently selected from the group consisting of hydrogen, halogen, cyano group, nitro, =O, =S, =CR²¹R²², -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-SR²¹, -C₀₋₄ alkylidene-S(O)₂R²¹, -C₀₋₄ alkylidene-S(O)R²¹, -C₀₋₄ alkylidene-S(O)₂NR²¹R²², -C₀₋₄ alkylidene-S(O)NR²¹R²², -C₀₋₄ alkylidene-C(O)R²¹, -C₀₋₄ alkylidene-C(O)OR²¹, -C₀₋₄ alkylidene-C(O)NR²¹R²², -C₀₋₄ alkylidene-NR²¹R²², -C₀₋₄ alkylidene-NR²¹C(O)R²², -C₀₋₄ alkylidene-NR²¹S(O)₂R²², -C₀₋₄ alkylidene-NR²¹S(O)R²², -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
R²¹ and R²² are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₁₋₄ alkylidene-OR²⁴, -C₁₋₄ alkylidene-OC(O)R²⁴, -C₁₋₄ alkylidene-SR²⁴, -C₁₋₄ alkylidene-S(O)₂R²⁴, -C₁₋₄ alkylidene-S(O)R²⁴, -C₁₋₄ alkylidene-S(O)₂NR²⁴R²⁵, -C₁₋₄ alkylidene-S(O)NR²⁴R²⁵, -C₁₋₄ alkylidene-C(O)R²⁴, -C₁₋₄ alkylidene-C(O)OR²⁴, -C₁₋₄ alkylidene-C(O)NR²⁴R²⁵, -C₁₋₄ alkylidene-NR²⁴R²⁵, -C₁₋₄ alkylidene-NR²⁴C(O)R²⁵, -C₁₋₄ alkylidene-NR²⁴S(O)₂R²⁵, -C₁₋₄ alkylidene-NR²⁴S(O)R²⁵, - C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
each R²⁶ is independently selected from the group consisting of hydrogen, halogen, cyano group, nitro, =O, =S, =CR²⁴R²⁵, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²⁴, -C₀₋₄ alkylidene-OC(O)R²⁴, -C₀₋₄ alkylidene-SR²⁴, -C₀₋₄ alkylidene-S(O)₂R²⁴, -C₀₋₄ alkylidene-S(O)R²⁴, -C₀₋₄ alkylidene-S(O)₂NR²⁴R²⁵, -C₀₋₄ alkylidene-S(O)NR²⁴R²⁵, -C₀₋₄ alkylidene-C(O)R²⁴, -C₀₋₄ alkylidene-C(O)OR²⁴, -C₀₋₄ alkylidene-C(O)NR²⁴R²⁵, -C₀₋₄ alkylidene-NR²⁴R²⁵, -C₀₋₄ alkylidene-NR²⁴C(O)R²⁵, -C₀₋₄ alkylidene-NR²⁴S(O)₂R²⁵, -C₀₋₄ alkylidene-NR²⁴S(O)R²⁵, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁷;
R²⁴ and R²⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl; and
each R²⁷ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl.

In some embodiments, the A ring is selected from the group consisting of: 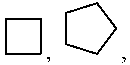 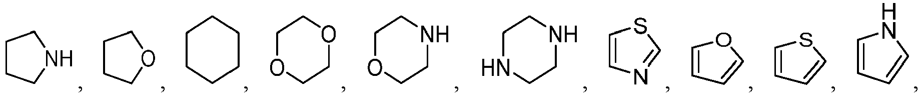 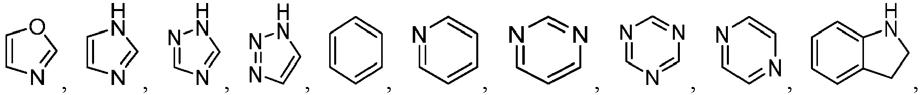 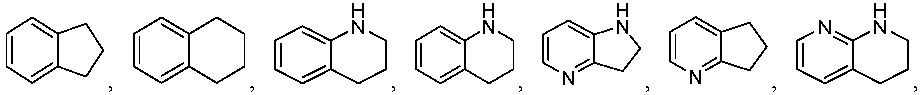 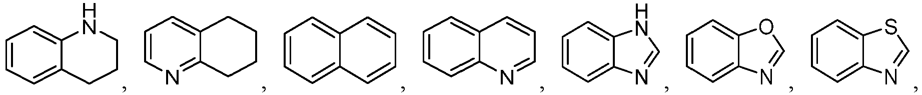 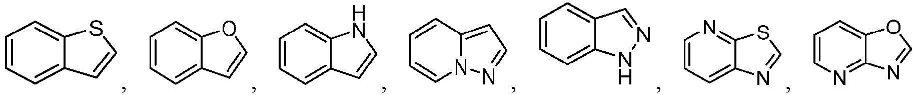 and 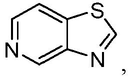 wherein the A ring is unsubstituted or substituted with one, two, three or four R^{A1}. In formula (I), R² and the carbonyl group connected to the A ring can be attached to any substitutable position of the A ring.

In some embodiments, each R^{A1} is independently selected from the group consisting of hydrogen, halogen, cyano group, nitro, =O, =S, -C₁₋₃ alkyl and halogen-substituted -C₁₋₃ alkyl.

In some embodiments, the A ring is selected from the group consisting of: 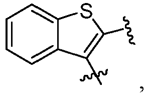 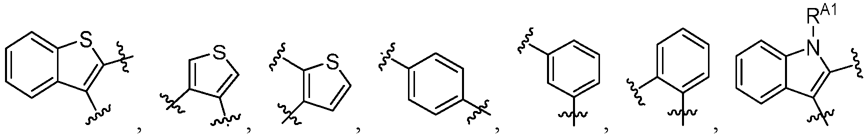 and

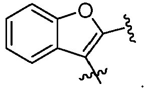

In some embodiments, R² is selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-C(O)R²¹, -C₀₋₄ alkylidene-C(O)OR²¹, -C₀₋₄ alkylidene-C(O)NR²¹R²², -C₀₋₄ alkylidene-NR²¹R²², -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²³;
each R²³ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-C(O)R²¹, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
R²¹ and R²² are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₁₋₄ alkylidene-OR²⁴, -C₁₋₄ alkylidene-OC(O)R²⁴, -C₁₋₄ alkylidene-C(O)R²⁴, -C₁₋₄ alkylidene-C(O)OR²⁴, -C₁₋₄ alkylidene-C(O)NR²⁴R²⁵, -C₁₋₄ alkylidene-NR²⁴R²⁵, -C₁₋₄ alkylidene-NR²⁴C(O)R²⁵, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
each R²⁶ is independently selected from the group consisting of hydrogen, halogen, cyano group, nitro, =O, =S, =CR²⁴R²⁵, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²⁴, -C₀₋₄ alkylidene-OC(O)R²⁴, -C₀₋₄ alkylidene-C(O)R²⁴, -C₀₋₄ alkylidene-C(O)OR²⁴, -C₀₋₄ alkylidene-C(O)NR²⁴R²⁵, -C₀₋₄ alkylidene-NR²⁴R²⁵, -C₀₋₄ alkylidene-NR²⁴C(O)R²⁵, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring); and
R²⁴ and R²⁵ are each independently selected from the group consisting of hydrogen, - C₁₋₃ alkyl and halogen-substituted -C₁₋₃ alkyl.

In some embodiments, R² is selected from the group consisting of: -C(O)NR²¹R²², -C(O)R²¹, -C_{0~2} alkylidene-NR²¹R²² and -C(O)OR²¹;
R²¹ and R²² are each independently selected from the group consisting of hydrogen, - C₁₋₃ alkyl, -C₀₋₁ alkylidene-(6-membered aromatic ring), -C₀₋₁ alkylidene-(10-membered heteroaromatic ring), -(4 to 6-membered heterocycloalkyl) and -(3 to 6-membered cycloalkyl), wherein aromatic ring, heteroaromatic ring, heterocycloalkyl and cycloalkyl are independently unsubstituted or substituted with one, two, three or four R²⁶;
each R²⁶ is selected from the group consisting of hydrogen, -C₁₋₃ alkyl, -(4 to 6-membered heterocycloalkyl), -C(O)R²⁴, -C(O)OR²⁴ and -OC(O)R²⁴; and
R²⁴ is independently selected from the group consisting of hydrogen, methyl and ethyl.

In some embodiments, R² is selected from the group consisting of: hydrogen, 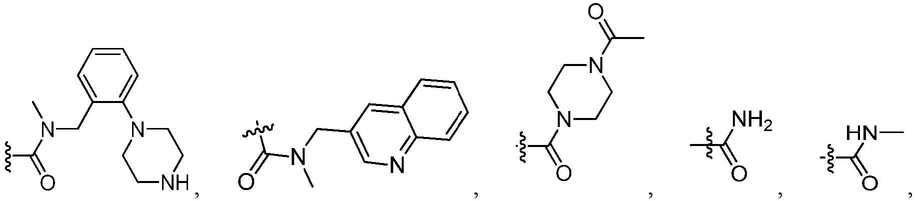 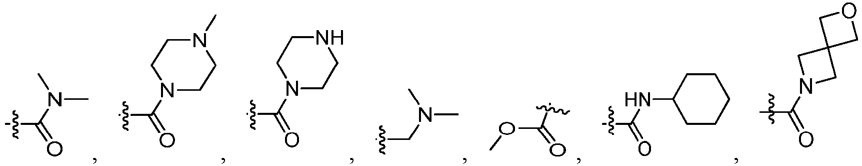 and

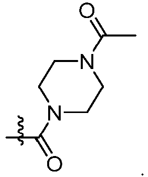

In some embodiments, the compound is selected from the group consisting of:

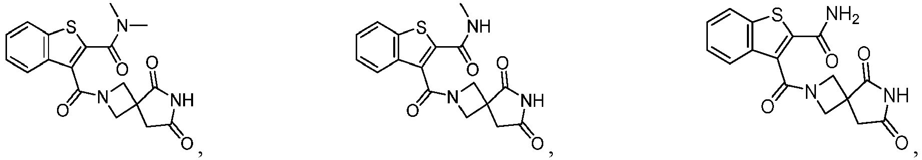

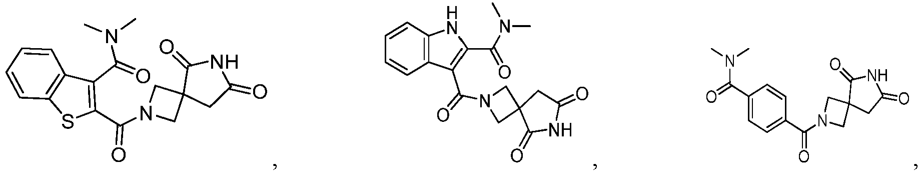

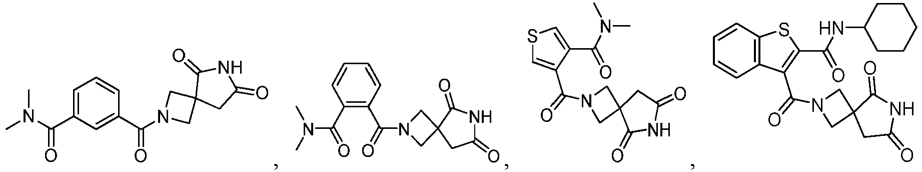

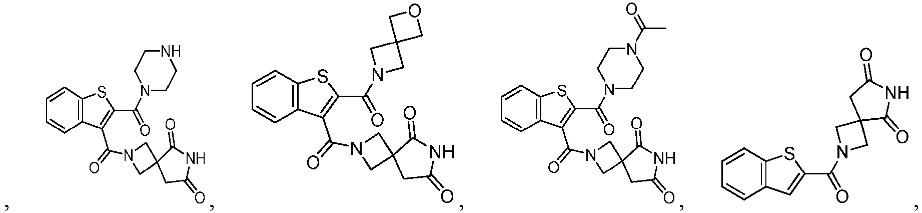

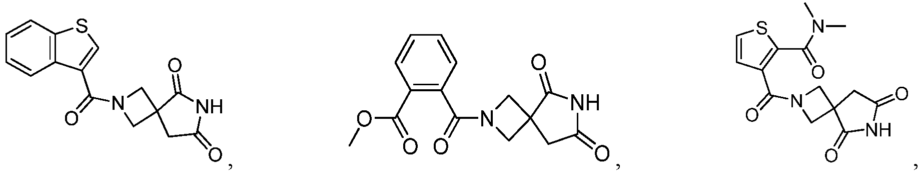

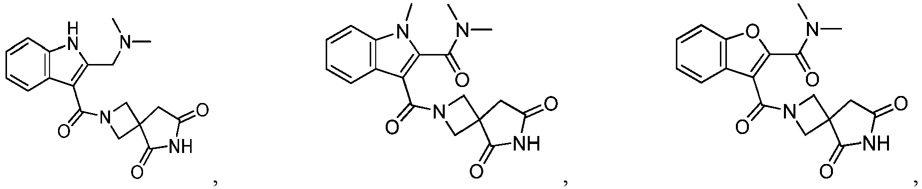

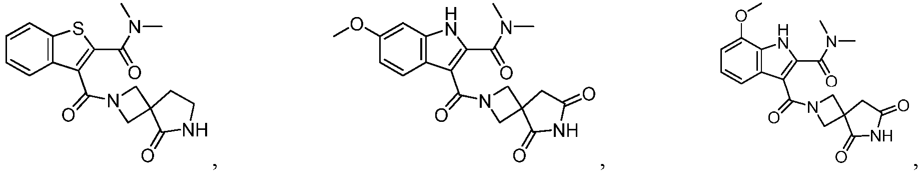

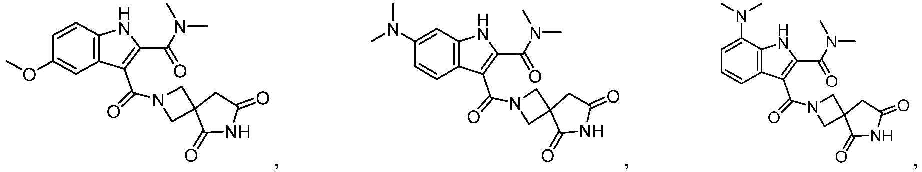

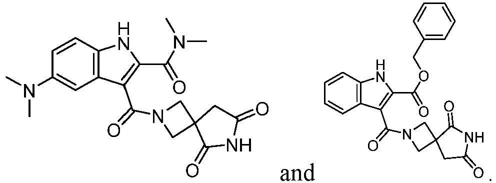

In a second aspect, this application provides a method for treating a disease associated with abnormal cell proliferation in a subject in need thereof, comprising:
administering to the subject a therapeutically effective amount of the above-mentioned compound of formula (I), or a stereoisomer, a deuterated compound or a pharmaceutically acceptable salt thereof.

In some embodiments, the disease is a cancer.

In a third aspect, this application provides a method for preparing a drug for targeted protein degradation, comprising:
preparing the drug for targeted protein degradation from the above-mentioned compound of formula (I), or a stereoisomer, a deuterated compound or a pharmaceutically acceptable salt thereof.

In a fourth aspect, this application provides a drug for targeted protein degradation, comprising:
the above-mentioned compound of formula (I), or a stereoisomer, a deuterated compound or a pharmaceutically acceptable salt thereof.

In some embodiments, the drug for targeted protein degradation is a drug that relies on a cereblon E3 ubiquitin ligase (CRBN) for protein degradation.

The compounds provided herein and derivatives thereof can be named according to the nomenclature system of the International Union of Pure and Applied Chemistry (IUPAC) or Chemical Abstracts Service (CAS), Columbus, Ohio.

Unless otherwise specified, the initial definition of group or term provided herein is applicable throughout the specification. And those terms that are not specifically defined herein should be construed according to the disclosure and context.

"Substitution" means that the hydrogen atom in the molecule is replaced by other different atoms or groups; or the lone electron pair of an atom in the molecule is replaced by S atom or O atom.

The limitation "capable of being substituted" indicates that a "substitution" may occur, but is not required. The description includes instances where it does or does not occur.

A minimum and a maximum of a content of carbon atoms in a hydrocarbon group are indicated by the prefix. For example, a prefix C_{a-b} alkyl indicates any alkyl group containing a-b carbon atoms, i.e., C₁₋₄ alkyl refers to an alkyl group containing 1-4 carbon atoms.

The "alkyl" refers to a saturated hydrocarbon chain having the specified number of member atoms, i.e., C₁₋₆ alkyl refers to an alkyl group containing 1-6 carbon atoms. The alkyl group can be straight-chain or branched. Representative branched alkyl groups have one, two or three branched chains. The alkyl group may optionally be substituted with one or more substituents as defined herein. The alkyl includes methyl, ethyl, propyl (n-propyl and isopropyl), butyl (n-butyl, isobutyl and tert-butyl), pentyl (n-pentyl, isopentyl and neopentyl) and hexyl. The alkyl group may also be part of other groups, such as C₁₋₆ alkoxy.

The term "alkylidene" in the present disclosure means a divalent saturated aliphatic hydrocarbon group having a specified number of carbon atoms. The "C_{a-b} alkylidene" refers to alkylidene groups with a-b carbon atoms. The alkylidene groups include both branched and straight-chain hydrocarbon groups. For example, C₁₋₆ alkylidene is intended to include methylene, ethylidene, propylidene, 2-methylpropylidene, dimethylethylidene and pentylidene. For example, propylidene is 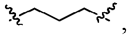 and dimethylbutylidene is 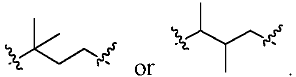 Moreover, the term "C₁₋₆ alkylidene" is also intended to include a branched hydrocarbon group, such as cyclopropyl methylene, exemplarily represented by 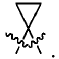 For example, the term "C₀₋₄ alkylidene" is alkylidene with C₀, alkylidene with C₁ (such as -CH₂-), alkylidene with C₂ (such as -CH₂CH₂-), alkylidene with C₃ (such as -CH₂CH₂CH₂-), or alkylidene with C₄ (such as -CH₂CH₂CH₂CH₂-). C₀ alkylidene refers to the absence of the group here, and a connection here is chemical bonding. For example, A-C₀ alkylidene-B refers to A-B, that is, A is directly connected to B through a chemical bond.

The term "alkenyl" refers to a straight or branched hydrocarbon group having a specified number of carbon atoms, 2-6 carbon atoms or 2-4 carbon atoms, and having at least one vinyl unsaturated site (>C=C<). For example, C_{a-b} alkenyl is an alkenyl group with a-b carbon atoms, such as vinyl, propenyl, isopropenyl and 1,3-butadienyl.

As used herein, the term "alkenylene" refers to a hydrocarbon chain having 2-10 carbon atoms, at least one double bond and two unsaturated valences. For example, (C₃-C₆) alkenylene includes ">C=CH-CH₂-" and "-CH₂-CH=CH-CH₂-".

The term "alkynyl" is a straight-chain monovalent hydrocarbon radical or a branched monovalent hydrocarbon radical containing at least one triple bond. The term "alkynyl" includes those alkyl groups having a triple bond and a double bond. For example, C₂₋₆ alkynyl includes ethynyl and propynyl.

The term "halogen" is fluorine, chlorine, bromine or iodine.

The terms "haloalkyl" and "halogen-substituted alkyl" refer to an yl in which the hydrogen atom may be replaced with one or more halogen atoms. For example, halogen-substituted C₁₋₄ alkyl refers to an alkyl group containing 1-4 carbon atoms with the hydrogen atoms substituted by one or more halogen atoms.

The terms "-OR" and "-NRR" in the present disclosure indicate that the R group is connected to the O atom or N atom by a single bond.

The terms "-C(O)R" and "-S(O)₂R" in the present disclosure indicate that the O atom is connected to the C atom or S atom by a double bond, and the R is connected to the C or S atom by a single bond.

The term "cycloalkyl" and "cycloalkane" in the present disclosure refer to a saturated or partially saturated cyclic group having a plurality of carbon atoms without heterocyclic atom, and having a single ring or a plurality of rings (including fused, bridged, spiro, and adamantane systems). For polycyclic systems with aromatic and non-aromatic rings that do not contain heterocyclic atoms, the term "cycloalkyl" is applicable when the attachment point is located at a non-aromatic carbon atom (for example, 5,6,7,8-tetrahydronaphthalen-5-yl). The terms "cycloalkyl" includes cycloalkenyl groups, such as cyclohexenyl. The cycloalkyl includes adamantyl, cyclopropyl, cyclobutyl, cyclohexyl, cyclopentyl, cyclooctyl, cyclopentenyl and cyclohexenyl. The cycloalkyl including a polybicycloalkyl ring systems includes dicyclohexyl, dicyclopentyl and dicyclooctyl, such as 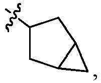 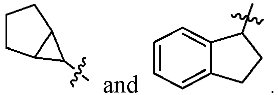 The adamantyl includes, but is not limited to, the following structure:

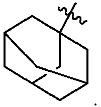

The terms "heterocycle", "heterocycloalkyl" and "heterocycloalkane" in the present disclosure refer to a saturated ring or a non-aromatic unsaturated ring containing at least one heteroatom, where the heteroatom refers to atoms such as nitrogen, oxygen and sulfur. For example, monovalent saturated or partially unsaturated monocyclic or bicyclic ring systems of a plurality of ring atoms, preferably a monovalent saturated or partially unsaturated single-ring or bicyclic ring system containing 3 to 9 ring atoms, which includes 1, 2, or 3 heteroatoms selected from the group consisting of N, O, and S, and the remaining ring atoms are carbon. Bicycles represent a chain consisting of two rings atoms in common, that is, a bridge separating the two rings is either a single bond or one or two ring atoms. The monocyclic saturated heterocyclicalkyl includes oxetanyl, azetidinyl, pyrrolidinyl, 2-oxopyrrolidin-3-yl, tetrahydrofuranyl, tetrahydrothienyl, pyrazolidinyl, imidazolidinyl, thiazolidinyl, piperidinyl, tetrahydropyranyl, tetrahydrothiopyranyl, piperazinyl, morpholinyl, 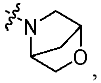 thiomorpholinyl, 1,1-dioxo-thiomorpholin-4-yl, azepanyl, diazepanyl, homopiperazinyl and oxazepanyl. The bicyclic saturated heterocycloalkyl includes 8-azabicyclo[3.2.1]octan, quinuclidinyl, 8-oxa-3-azabicyclo[3.2.1]octan, 9-azabicyclo[3.3.1]nonane and 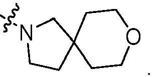 The partially unsaturated heterocycloalkyl includes dihydrofuranyl, imidazolinyl, tetrahydro-pyridyl and dihydropyranyl.

The terms "spiro-heterocyclic group" and "spiro-heterocycle" can be used interchangeably and refer to a non-aromatic saturated ring or a non-aromatic unsaturated ring system in which two monocycles share a carbon atom, consisting of carbon atoms and heteroatoms selected from the group consisting of N, O, S and P. For example, "5 to 12-membered spiro-heterocycle" refers to a spiro-heterocycle having 5 to 12 ring atoms, where 1, 2 or 3 of the ring atoms are heteroatoms.

The terms "bridged-ring" or "bridged-ring group" refer to a saturated or unsaturated cyclic group formed by two or more cyclic structures sharing two non-adjacent atoms with each other, including but not limited to

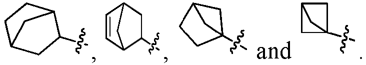

The terms "bridged-heterocyclic group" and "bridged-heterocycle" can be used interchangeably and refer to a saturated or unsaturated cyclic group formed by two or more cyclic structures sharing two non-adjacent atoms with each other, composed of carbon atoms and heteroatoms selected from the group consisting of N, O, S, and P. Specific examples include, but are not limited to

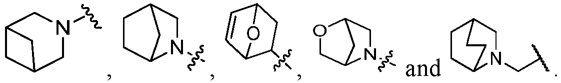

The terms "aromatic ring" and "aryl group" mentioned herein refer to an aromatic group with a plurality of carbon atoms. The aryl group is usually a monocyclic, bicyclic or tricyclic aryl having 5-20 carbon atoms. Furthermore, the term "aryl group" provided herein refers to an aromatic substituent that can be a single aromatic ring or multiple aromatic rings fused together. Non-limiting examples include phenyl, naphthyl and tetrahydronaphthyl.

The terms "heteroaromatic ring" and "heteroaryl group" mentioned herein refer to an aromatic unsaturated ring containing at least one heteroatom. The heteroatom includes nitrogen atoms, oxygen atoms and sulfur atoms. For example, aromatic monocyclic or bicyclic hydrocarbons with a plurality of ring atoms, where one or more of the ring atoms are selected from the group consisting of O, N and S. Preferably, there are 1-3 heteroatoms. The heteroaryl group includes pyridyl, indolyl, quinoxalinyl, quinolinyl, isoquinolinyl, benzothienyl, benzofuranyl, benzopyranyl, benzothiopyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl and benzoxazolyl.

The term "stereoisomer" mentioned herein includes enantiomer and diastereomers.

The term "deuterated compound" mentioned herein refers to a substitution of one or more hydrogen atoms in a molecule or group by deuterium atoms, where the percentage of deuterium atoms is greater than an abundance of deuterium in nature.

The term "pharmaceutically acceptable" mentioned herein refers to a carrier, delivery agent, diluent, excipient, and/or salt that are generally chemically or physically compatible with other ingredients used in a pharmaceutical dosage form, and are physiologically compatible with the receptor.

The terms "salt" and "pharmaceutically acceptable salt" mentioned herein refer to salts formed by the aforementioned compounds or their stereoisomers with inorganic and/or organic acids and bases, including both acidic and/or base salts, as well as zwitterionic salts (inner salts) and quaternary ammonium salts, such as alkylammonium salts. These salts can be directly obtained during the final separation and purification of the compound. Alternatively, they can also be obtained by mixing the aforementioned compounds or their stereoisomers with an appropriate amount of acid or base (e.g., equivalent). The salts may be insoluble, and can be collected by filtration, evaporation or freeze drying. The salts provided herein include but not limited to hydrochloride, sulfate, citrate, benzene sulfonate, hydrobromide, hydrofluoride, phosphate, acetate, propionate, succinate, oxalate, malate, succinate, fumarate, maleate, tartarate and trifluoroacetate.

Apparently, according to the foregoing disclosure, various modifications, replacements or variations can be made by those skilled in the art without departing from the spirit of the present disclosure.

### DETAILED DESCRIPTION OF EMBODIMENTS

The disclosure will be further described in detail below with reference to the following examples. However, it should be understood that the scope of the present disclosure is not limited to the following exemplary embodiments. All embodiments implemented in light of the content disclosed herein shall fall within the scope of the present disclosure.

The known starting materials of the present disclosure can be synthesized according to the methods known in the prior art, or can be purchased from Energy Chemical Co., Ltd, Chengdu Kelong Chemical Co., Ltd, Accela ChemBio Co., Ltd, or J&K Scientific Co., Ltd. DIPEA: N,N-diisopropylethylamine; HATU: 2-(7-azabenzotriazol-1-yl)-N,N,N',N'-tetramethyluronium hexafluorophosphate; HOBT: 1-hydroxybenzotriazole; DMSO: dimethyl sulfoxide; LC-MS: liquid chromatograph mass spectrometry; NaCl: sodium chloride; MPLC: medium pressure preparative liquid chromatography; EDCI: 1-(3-dimethylaminopropyl)-3-ethylcarbodiimide hydrochloride; Pd(OAc)₂: palladium (II) acetate; DMF: N,N-dimethylformamide; AIBN: 2,2'-Azobis(2-methylpropionitrile); NBS: N-bromosuccinimide; and BPO: dibenzoyl peroxide.

Unless otherwise specified, reaction was carried out under a nitrogen atmosphere, a solution is an aqueous solution, a temperature of the reaction is room temperature, which is the most suitable temperature for the reaction (20°C to 30°C), and M represents mol/L.

Compounds are structurally characterized by Nuclear Magnetic Resonance (NMR) and Mass Spectrometry (MS). The NMR shifts (δ) is expressed in a unit of 10⁻⁶ (ppm). The NMR spectra are obtained by using a Nuclear Magnetic Resonance Spectrometer (Bruker Avance III 400) and (Bruker Avance 600 spectrometer) with deuterated dimethyl sulfoxide (DMSO-*d₆*), deuterated chloroform (CDCl₃), or deuterated methanol (Methol-*d₄*) as the solvent, and tetramethylsilane (TMS) as the internal standard. LC-MS is carried out using Shimadzu LC-MS 2020 (ESI). HPLC is performed using a Shimadzu High-Performance Liquid Chromatograph (Shimadzu LC-20A). MPLC (Medium-Pressure Preparative Liquid Chromatography) is performed on a Gilson GX-281 reversed phase preparative chromatograph. Yantai Huanghai HSGF254 or Qingdao GF254 silica gel plates are used for thin layer chromatography, and the product size used in the thin layer chromatography is 0.4 mm to 0.5 mm. Typically, 200-300 mesh silica gel (Yantai Huanghai silica gel) is used as the carrier in column chromatography.

### EXAMPLE 1 Preparation of compound A1

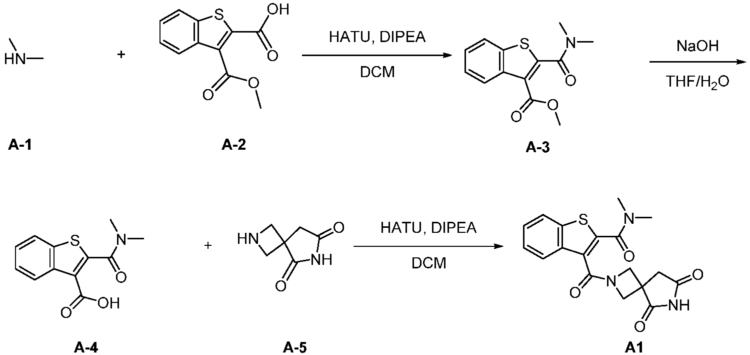

### (1) Preparation of compound A-3

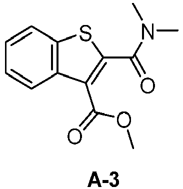

Compound **A-2** (50.00 mg, 211.65 µmol), DIPEA (132.25 mg, 0.80 mmol, 178.06 µL), HATU (250.96 mg, 0.66 mmol) and dichloromethane (2 mL) were successively added to a 50 mL reaction flask. After the temperature of the reaction mixture was lowered to 0 °C, compound **A-1** (86.29 mg, 1.06 mmol) was added. The reaction mixture was reacted under stirring in an ice bath for 0.5 h, and quenched (monitored by LC-MS), and then added with a saturated NaCl solution (10 mL) and ethyl acetate (3×20 mL) for extraction. The organic phases were combined, dried over anhydrous sodium sulfate, concentrated under reduced pressure to obtain the compound **A-3** (crude product).

### (2) Preparation of compound A-4

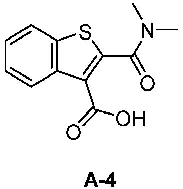

Compound **A-3** (crude product), sodium hydroxide (47.95 mg, 854.50 mmol), tetrahydrofuran (4 mL) and water (2 mL) were successively added to a 50 mL reaction flask. The reaction mixture was reacted under stirring at room temperature for 8 h, quenched (monitored by LC-MS), adjusted to pH 6.0-7.0 with a 1N hydrochloric acid solution and concentrated to give the compound **A-4** (crude product), which was used directly in the next step.

### (3) Synthesis of compound A1

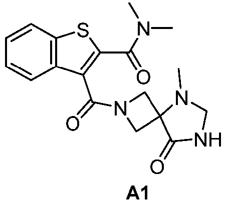

Compound **A-4** (8.70 mg, 34.90 µmol), EDCI (13.33 mg, 69.80 µmol), HOBT (9.43 mg, 69.80 µmol), DIPEA (11.28 mg, 87.25 µmol, 15.20 µL) and DMSO (1 mL) were successively added to a 50 mL reaction flask. The reaction mixture was reacted at room temperature for 10 min, added with compound **A-5** (4.89 mg, 34.90 µmol), reacted under stirring at room temperature for 1 h, and quenched (monitored by LC-MS). Then the reaction mixture was added with a saturated NaCl solution (10 mL) and ethyl acetate (3 × 20 mL) for extraction. The organic phases were combined, dried over anhydrous sodium sulfate, concentrated under reduced pressure and purified by medium-pressure liquid chromatography (MPLC) to give the compound **A1** (2.30 mg, 6.20 µmol, 17.12% yield, 81.1% purity).

LC-MS: C₁₈H₁₈N₃O₄S, [M+H]+ 372.1; found 372.2.

¹H NMR (400 MHz, Methanol-*d4*) δ7.98-7.95 (m, 2H), 7.54-7.49 (m, 2H), 4.44-4.05 (m, 4H), 3.21 (s, 3H), 3.13 (s, 3H), 3.03-2.99 (m, 2H).

Referring to the synthesis method of compound **A1,** compound **A-1** and compound **A-2** were respectively replaced with the raw material 1 and raw material 2 listed in the following table (other reagents and experimental conditions remained unchanged) to yield compounds **A2-A11.**

| Compou nd number | Compound structure and characterization data | Raw material 1 | Raw material 2 | Yield |
|---|---|---|---|---|
| **A2** | 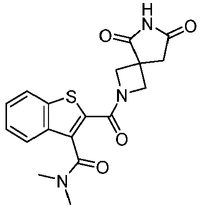 Compound **A2** (7.50 mg, 20.19 µmol, 25.17% yield, 96.7% purity). LC-MS:C₁₈H₁₈N₃O₄S, [M+H]⁺ 372.1; found 372.1. ¹H NMR (600 MHz, DMSO-*d*₆) δ | 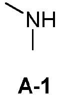 | 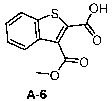 | 25.17% |
| | 11.26 (s, 1H), 8.12 (d, *J =* 7.8 Hz, 1H), 7.66 (d, *J =* 7.8 Hz, 1H), 7.53 (m, 2H), 4.51 (s, 1H), 4.39 (s, 1H), 4.21 (s, 1H), 4.07 (s, 1H), 3.07 (s, 3H), 3.00 (s, 2H), 2.75 (s, 3H). | | | |
| **A3** | 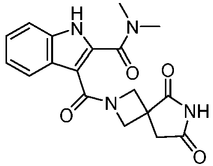 Compound **A3** (2.70 mg, 7.62 µmol, 8.85% yield, 98.1% purity). LC-MS:C₁₈H₁₉N₄O₄, [M+H]⁺ 355.1; found 355.1. ¹H NMR (400 MHz, DMSO-*d*₆) δ 12.09 (s, 1H), 11.19 (s, 1H), 7.20 (m, 2H), 4.16 (d, *J =* 9.6 Hz, 2H), 4.00 (d, *J* = 9.6 Hz, 2H), 3.06 (s, 3H), 2.97 (m, 5H). | 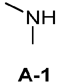 | 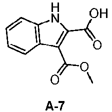 | 8.85% |
| **A4** | 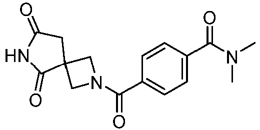 Compound **A4** (2.00 mg, 6.34 µmol, 6.13% yield, 90.9% purity). LC-MS: C₁₆H₁₈N₃O₄, [M+H]⁺ 316.1; found 316.2. ¹H NMR (400 MHz, DMSO-*d*₆) δ 10.97 (s, 1H), 7.80-7.59 (m, 2H), 7.56-7.35 (m, 2H), 4.45-4.31 (m, 2H), 4.23 (mz, 1H), 4.06 (m, 1H), 2.98 (m, 5H), 2.89 (s, 3H). | 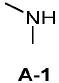 | 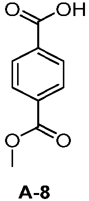 | 6.13% |
| **A5** | 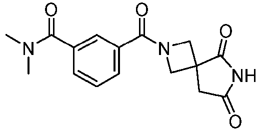 Compound **A5** (4.30 mg, 13.64 µmol, 42.22% yield, 95.9% purity). LC-MS: C₁₆H₁₈N₃O₄, [M+H]⁺ 316.1; found 316.1. ¹H NMR (400 MHz, Chloroform-*d*) δ 7.79-7.60 (m, 2H), 7.58-7.42 (m, 2H), 4.86-4.44 (m, 2H), 4.33-4.00 (m, 2H), 3.20-2.89 (m, 8H). | 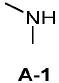 | 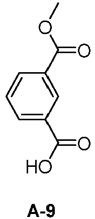 | 42.22% |
| **A6** | 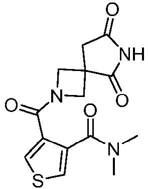 Compound **A6** (8.00 mg, 24.89 µmol, 24.80% yield, 95.3% purity). LC-MS: C₁₄H₁₆N₃O₄S, [M+H]⁺ 322.1; found 322.1. ¹H NMR (400 MHz, DMSO-*d*₆) δ 11.24 (s, 1H), 7.97 (d, *J* = 2.8 Hz, 1H), 7.63 (d, *J =* 2.8 Hz, 1H), 4.42 (m, 1H), 4.29 (m, 1H), 4.16 (m, 1H), 3.98 (m, 1H), 3.03-2.74 (m, 8H). | 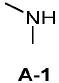 | 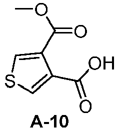 | 24.80% |
| **A7** | 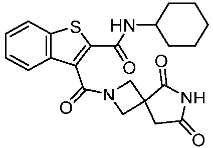 Compound **A7** (2.20 mg, 5.17 µmol, 5.60% yield, 99.1% purity). LC-MS: C₂₂H₂₄N₃O₄S₄, [M+H]⁺ 426.1; found 426.1 ¹H NMR (400 MHz, DMSO-*d*₆) δ 8.15-8.03 (m, 1H), 7.94-7.80 (m, 1H), 7.63-7.45 (m, 2H), 4.33 (d, *J =* 10.3 Hz, 1H), 4.19 (d, *J* = 10.3 Hz, 1H), 3.97-3.85 (m, 2H), 3.78 (m, 1H), 3.04-2.83 (m, 2H), 1.86 (s, 2H), 1.71 (s, 2H), 1.57 (m, 1H), 1.29 (m, 5H). | 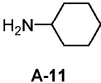 | 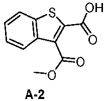 | 5.60% |
| **A8** | 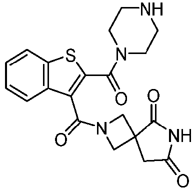 Compound **A8** (4.10 mg, 9.95 µmol, 12.32% yield, 95.1% purity). LC-MS: C₂₀H₂₁N₄O₄S, [M+H]⁺ 413.1; found 413.4 ¹H NMR (400 MHz, DMSO-*d*₆) δ 11.26 (s, 1H), 9.37 (s, 2H), 8.18-8.04 (m, 1H), 7.96-7.81 (m, 1H), 7.61-7.47 (m, 2H), 4.31-4.02 (m, 3H), 3.91 (m, 1H), 3.73-3.60 (m, 4H), 3.17 (m, 4H), 2.94 (m, 2H). | 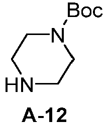 | 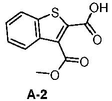 | 12.32% |
| **A9** | 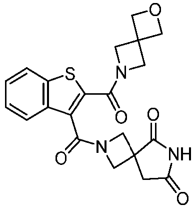 Compound **A9** (2.60 mg, 6.11 µmol, 10.30% yield, 93.0% purity). LC-MS: C₂₁H₂₀SO₅N, [M+H]⁺ 426.5; found 426.6 ¹H NMR (400 MHz, DMSO-d₆) δ 11.24 (s, 1H), 8.30-7.96 (m, 1H), 7.98-7.67 (m, 1H), 7.54-7.30 (m, 1H), 4.47 (s, 2H), 4.34-4.20 (m, 3H), 4.13 (d, J *=* 10.2 Hz, 1H), 3.99 (d, J = 9.0 Hz, 1H), 3.86 (d, J = 9.0 Hz, 1H), 3.06-2.80 (m, 2H). | 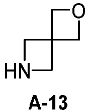 | 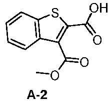 | 10.30% |
| **A10** | 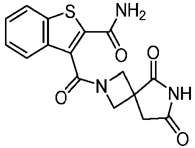 Compound **A10** (1.30 mg, 3.79 µmol, 3.35% yield, 93.0% purity). LC-MS: C₁₆H₁₄SO₄N₃, [M+H]⁺ 344.1; found 344.0 ¹H NMR (400 MHz, DMSO-*d*₆) δ 8.17-8.03 (m, 1H), 7.86-7.77 (m, 1H), 7.55-7.45 (m, 2H), 4.42-4.13 (m, 2H), 3.92-3.76 (m, 2H), 3.04-2.80 (m, 2H). | NH₄Cl | 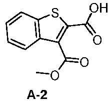 | 3.35% |
| | | **A-14** | | |
| **A11** | 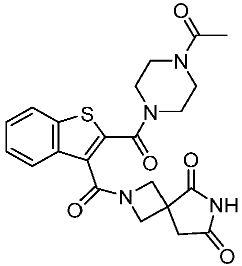 Compound **A11** (6.00 mg, 13.20 µmol, 14.63% yield, 86.8% purity). LC-MS: C₂₂H₂₃SO₅N₄, [M+H]⁺ 455.1; found 455.0 ¹H NMR (400 MHz, DMSO-d₆) 1H NMR (400 MHz, DMSO-d6) δ 11.22 (s, 1H), 8.24-7.99 (m, 1H), 8.01-7.72 (m, 1H), 7.69-7.30 (m, 2H), 4.52-3.85 (m, 4H), 3.80-3.60 (m, 6H), 3.02-2.88 (m, 4H), 2.04 (s, 3H). | 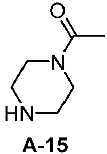 | 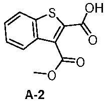 | 14.63% |

### EXAMPLE 2 Preparation of compound A16

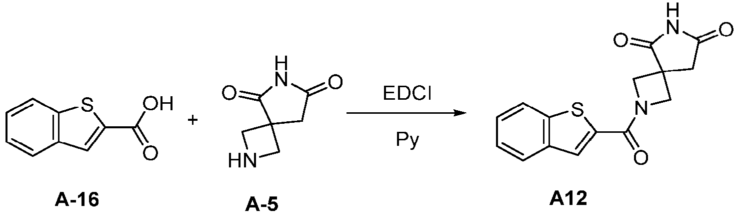

### (1) Synthesis of compound A12

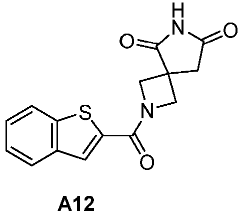

Compound **A-16** (20.00 mg, 112.23 µmol), **A-5** (15.73 mg, 112.23 µmol), EDCI (42.87 mg, 224.46 µmol) and pyridine (2 mL) were successively added to a 50 mL reaction flask. The reaction mixture was reacted under stirring in an ice bath for 0.5 h, quenched (monitored by LC-MS), and then added with a saturated NaCl solution (10 mL) and ethyl acetate (3 × 20 mL) for extraction. The organic phases were combined, dried over anhydrous sodium sulfate, purified by medium-pressure liquid chromatography (MPLC) and concentrated under reduced pressure to give the compound **A12** (10.00 mg, 33.30 µmol, 29.67% yield, 99.9% purity).

LC-MS: C₁₅H₁₃N₂O₃S, [M+H]⁺ 301.1; found 301.2.

¹H NMR (600 MHz, DMSO-d₆) δ 11.29 (s, 1H), 8.04 (d, *J =* 7.8 Hz, 1H), 7.96 (d, *J =* 7.8 Hz, 1H), 7.86 (s, 1H), 7.51-7.44 (m, 2H), 4.71 (d, *J =* 9.0 Hz, 1H), 4.61 (d, *J =* 9.0 Hz, 1H), 4.25 (m, 1H), 4.11 (m, 1H), 3.03 (s, 2H).

Referring to the synthesis method of compound A12, compound A-16 was replaced with the raw material 1 listed in the following table (other reagents and experimental conditions remained unchanged) to yield compounds A13-A14.

| Compound number | Compound structure and characterization data | Raw material 1 | Yield |
|---|---|---|---|
| **A13** | 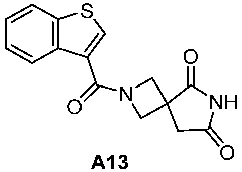 Compound **A13** (6.00 mg, 19.98 µmol, 17.80% yield, purity 99.9%). LC-MS: C₁₅H₁₃N₂O₃S, [M+H]⁺ 301.1; found 301.2. ¹H NMR (600 MHz, DMSO-*d*₆) δ 11.24 (s, 1H), 8.37-8.21 (m, 2H), 8.06 (d, *J =* 7.8 Hz, 1H), 7.60-7.35 (m, 2H), 4.47 (d, *J =* 9.0 Hz, 1H), 4.37 (d, *J =* 9.0 Hz, 1H), 4.26 (m, 1H), 4.10 (m, 1H), 3.01 (m, 2H). | 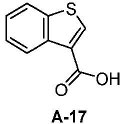 | 17.80% |
| **A14** | 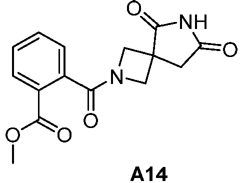 Compound **A14** (17.00 mg, 50.84 µmol, 14.26% yield, 90.5% purity). LC-MS: C₁₅H₁₅N₂O₅, [M+H]⁺ 303.1; found 303.3 ¹H NMR (400 MHz, DMSO-d₆) δ 7.87 (m, 1H), 7.62 (m, 2H), 7.43-7.36 (m, 1H), 4.20 (m, 1H), 4.01 (m, 2H), 3.87 (s, 4H), 3.06-2.88 (m, 2H). | 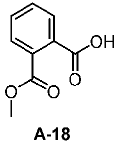 | 14.26% |

### EXAMPLE 3 Preparation of compound A15

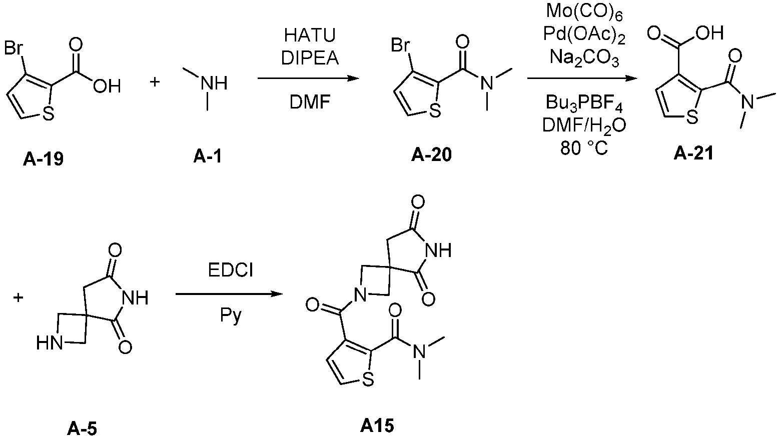

### (1) Synthesis of compound A-20

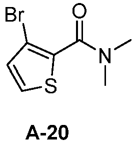

Compound **A-19** (155.00 mg, 0.75 mmol), DIPEA (132.25 mg, 1.00 mmol, 178.06 µL), HATU (250.96 mg, 0.66 mmol) and dichloromethane (2 mL) were successively added to a 50 mL reaction flask. After the temperature of the reaction system was decreased to 0 °C, compound **A-1** (190.00 mg, 0.75 mmol) was added. The reaction mixture was reacted under stirring in an ice bath for 0.5 h, quenched (monitored by LC-MS), and then added with a saturated NaCl solution (10 mL) and ethyl acetate (3 × 20 mL) for extraction. The organic phases were combined, dried over anhydrous sodium sulfateand concentrated under reduced pressure to obtain the compound **A-20** (100.00 mg, crude product).

### (2) Synthesis of compound A-21

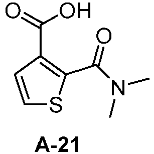

Compound **A-20** (117.00 mg, 0.50 mmol), sodium carbonate (91.00 mg, 1.00 mmol), Mo(CO)₆ (169.30 mg, 0.75 mmol), Pd(OAc)₂ (9.6 mg, 0.05 mmol) and Bu₃PBF₄ (24.73 mg, 0.10 mmol) were dissolved in DMF/H₂O (10 mL, 1:1) under a nitrogen atmosphere. The reaction mixture was reacted at 85 °C for 2 h, concentrated and purified by medium-pressure liquid chromatography (MPLC) to give the compound A-21 (70.00 mg, 70.35% yield).

### (3) Synthesis of compound A15

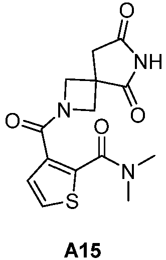

Compound **A-21** (20.00 mg, 100.39 µmol), compound **A-5** (14.07 mg, 100.39 µmol), EDCI (19.17 mg, 100.39 µmol) and pyridine (2 mL) were successively added to a 50 mL reaction flask. The reaction mixture was reacted under stirring in an ice bath for 0.5 h, quenched (monitored by LC-MS), and then added with a saturated NaCl solution (10 mL) and ethyl acetate (3 × 20 mL) for extraction. The organic phases were combined, dried over anhydrous sodium sulfate, purified by medium-pressure liquid chromatography (MPLC) and concentrated under reduced pressure to give the compound A15 (5.00 mg, 15.56 µmol, 15.50% yield, 99.9% purity).

LC-MS: C₁₄H₁₆N₃O₄S, [M+H]⁺ 322.1; found 322.1.

¹H NMR (400 MHz, DMSO-d₆) δ 11.23 (s, 1H), 7.72 (d, *J =* 5.2 Hz, 1H), 7.21 (d, *J =* 5.2 Hz, 1H), 4.39-4.10 (m, 3H), 4.00 (m, 1H), 3.08-2.76 (m, 8H).

### EXAMPLE 4 Preparation of compound A16

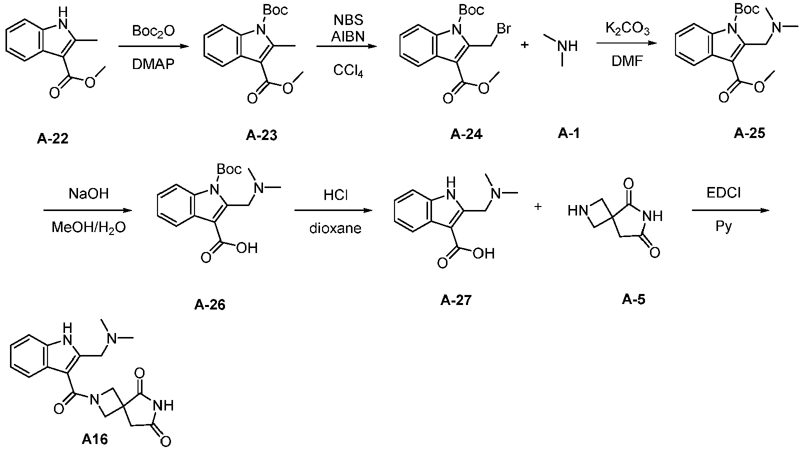

### (1) Synthesis of compound A-23

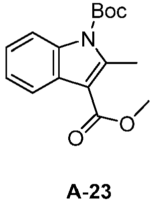

Compound **A-22** (500.00 mg, 2.64 mmol), DMAP (322.83 mg, 2.64 mmol) and Boc₂O (8.64 g, 39.64 mmol) were successively added to a 50 mL reaction flask. The reaction mixture was reacted under stirring overnight at room temperature, concentrated and purified by medium-pressure liquid chromatography (MPLC) to give the compound **A-23** (700.00 mg, 2.42 mmol, 91.56% yield).

### (2) Synthesis of compound A-24

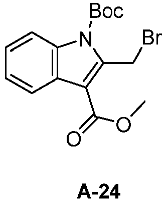

Compound **A-23** (700.00 mg, 2.42 mmol), NBS (452.15 mg, 2.54 mmol), AIBN (79.46 mg, 483.88 µmol) and carbon tetrachloride (7 mL) were successively added to a 50 mL reaction flask. The reaction mixture was refluxed overnight, concentrated and purified by medium-pressure liquid chromatography (MPLC) to give the compound **A-24** (700.00 mg, 1.90 mmol, 78.57% yield).

### (3) Synthesis of compound A-25

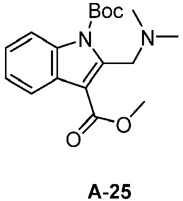

Compound **A-1** (36.73 mg, 814.73 µmol), compound **A-24** (100.00 mg, 271.58 µmol), potassium carbonate (187.39 mg, 1.36 mmol) and DMF (2 mL) were successively added to a 50 mL reaction flask. The reaction mixture was reacted under stirring at 60 °C for 2 h and purified by medium-pressure liquid chromatography (MPLC) to give the compound **A-25** (50.00 mg, 150.42 µmol, 55.39% yield).

### (4) Synthesis of compound A-26

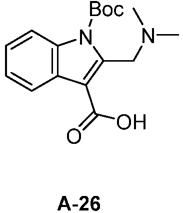

Compound **A-25** (50.00 mg, 150.42 µmol), sodium hydroxide (60.17 mg, 1.50 mmol) and MeOH/H₂O (2 mL, 1:1) were successively added to a 50 mL reaction flask. The reaction mixture was refluxed under stirring for 2 h, adjusted the pH with a 1 M HCl aqueous solution, concentrated and purified by medium-pressure liquid chromatography (MPLC) to give the compound **A-26** (45.00 mg, 141.35 µmol, 93.97% yield).

### (5) Synthesis of compound A-27

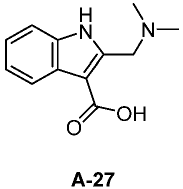

Compound **A-26** (45.00 mg, 141.35 µmol) was dissolved in hydrochloric acid/dioxane (2 mL). The reaction mixture was reacted under stirring at room temperature for 2 h and concentrated to give the compound **A-27** (30.00 mg, 137.46 µmol, 97.25% yield).

### (6) Synthesis of compound A16

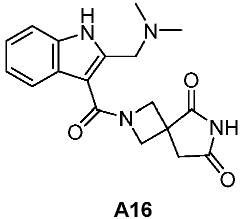

Compound **A-27** (45.00 mg, 206.18 µmol), compound **A-5** (28.89 mg, 206.18 µmol), EDCI (78.97 mg, 412.37 µmol) and pyridine (2 mL) were successively added to a 25 mL reaction flask. The reaction mixture was reacted under stirring in an ice bath for 0.5 h, quenched (monitored by LC-MS), and then added with a saturated NaCl solution (10 mL) and ethyl acetate (3 × 20 mL) for extraction. The organic phases were combined, dried over anhydrous sodium sulfate, purified by medium-pressure liquid chromatography (MPLC) and concentrated under reduced pressure to give the compound **A16** (8.60 mg, 25.27 µmol, 12.25% yield, 98.1% purity).

LC-MS: C₁₈H₂₁N₄O₃, [M+H]⁺ 341.2; found 341.1.

¹H NMR (400 MHz, DMSO-*d*₆) δ 7.53 (m, 1H), 7.42 (dd, *J* = 6.6, 2.4 Hz, 1H), 7.14 (m, 2H), 4.17 (d, *J =* 8.3 Hz, 2H), 4.01 (d, *J* = 9.2 Hz, 2H), 3.78 (s, 2H), 2.97 (s, 2H), 2.22 (s, 6H). Referring to the synthesis method of compound A16, compound A-22 was replaced with the raw material listed in the following table (other reagents and experimental conditions remained unchanged) to yield compound A17.

| Compoun d number | Compound structure and characterization data | Raw material | Yield |
|---|---|---|---|
| **A17** | 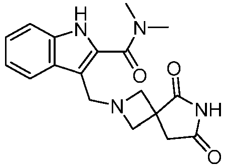 Compound **A17** (4.47 mg, 13.13 µmol, 28.92% yield, 98.1% purity). LC-MS: C₁₈H₂₁N₄O₃, [M+H]⁺ 341.2; found 341.1. ¹H NMR (400 MHz, DMSO-*d*₆) δ 12.04 (m, 1H), 7.85 (m, 1H), 7.51 (m, 1H), 7.32 (m, 1H), 7.22 (t, *J =* 7.6 Hz, 1H), 4.63 (m, 2H), 4.31-4.08 (m, 4H), 3.06 (s, 6H), 2.94 (m, 2H). | 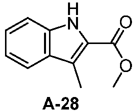 | 28.92% |

### EXAMPLE 5 Preparation of compound A18

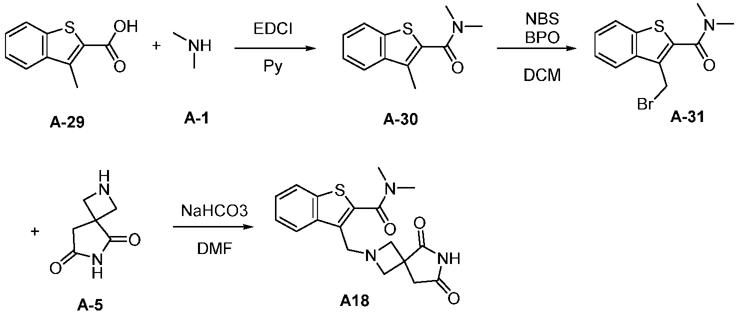

### (1) Synthesis of compound A-30

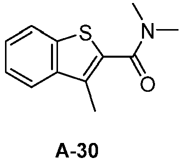

Compound **A-29** (1.00 g, 5.20 mmol), compound **A-1** (703.57 mg, 15.61 mmol), EDCI (2.99 g, 15.61 mmol) and pyridine (10 mL) were successively added to a 25 mL reaction flask. The reaction mixture was reacted under stirring in an ice bath for 0.5 h, quenched (monitored by LC-MS), and then added with a saturated NaCl solution (10 mL) and ethyl acetate (3 × 20 mL) for extraction. The organic phases were combined, dried over anhydrous sodium sulfate, purified by medium-pressure liquid chromatography (MPLC) and concentrated under reduced pressure to give the compound **A-30** (1.13 g, 5.15 mmol, 99.05% yield).

### (2) Synthesis of compound A-31

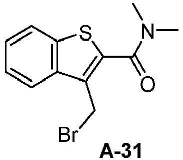

Compound **A-30** (100.00 mg, 455.99 µmol), BPO (22.09 mg, 91.20 µmol), NBS (85.22 mg, 478.79 µmol) and dichloromethane (DCM, 5 mL) were successively added to a 25 mL reaction flask. The reaction mixture was reacted under stirring overnight and purified by medium-pressure liquid chromatography (MPLC) to give the compound **A-31** (115.00 mg, 385.65 µmol, 84.57% yield).

### (3) Synthesis of compound A18

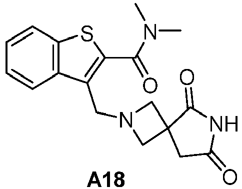

Compound **A-31** (45.00 mg, 150.91 µmol), compound **A-5** (21.15 mg, 150.91 µmol), sodium bicarbonate (63.38 mg, 754.53 µmol) and DMF (1 mL) were successively added to a 5 mL reaction flask. The reaction mixture was reacted under stirring overnight at room temperature and purified by medium-pressure liquid chromatography (MPLC) to give the compound **A-18** (17.85 mg, 49.94 µmol, 33.09% yield, 98.1% purity).

LC-MS: C₁₈H₂₁N₄O₃, [M+H]⁺ 341.2; found 341.1.

¹H NMR (400 MHz, DMSO-*d*₆) δ 7.53 (m, 1H), 7.42 (dd, *J =* 6.6, 2.4 Hz, 1H), 7.14 (m, 2H), 4.17 (d, *J* = 8.3 Hz, 2H), 4.01 (d, *J =* 9.2 Hz, 2H), 3.78 (s, 2H), 2.97 (s, 2H), 2.22 (s, 6H).

### EXAMPLE 6 Preparation of compound A19

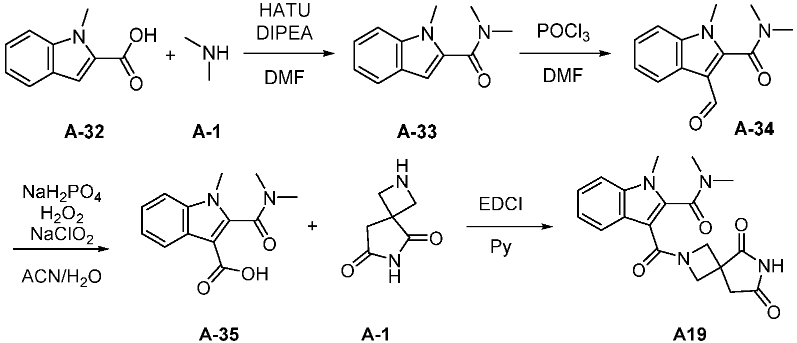

### (1) Synthesis of compound A-33

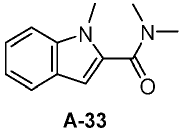

Compound **A-32** (500.00 mg, 2.85 mmol), HATU (1.08 g, 2.85 mmol), DIPEA (368.88 mg, 2.85 mmol, 497.14 µL) and dichloromethane (10 mL) were successively added to a 50 mL reaction flask. The reaction mixture was reacted under stirring in an ice bath for 20 min, followed by addition of the compound **A-1** (128.67 mg, 2.85 mmol). Then the reaction mixture was reacted under stirring at room temperature for 1 h, concentrated and purified by medium-pressure liquid chromatography (MPLC) to give the compound **A-33** (500.00 mg, 2.47 mmol, 86.62% yield).

### (2) Synthesis of compound A-34

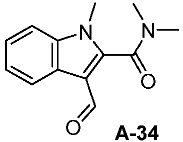

Compound **A-33** (500.00 mg, 2.47 mmol), phosphorus oxychloride (1.13 g, 7.41 mmol) and DMF (15 mL) were successively added to a 50 mL reaction flask. The reaction mixture was reacted under stirring at room temperature for 2 h and then purified by medium-pressure liquid chromatography (MPLC) to give the compound **A-34** (430.00 mg, 1.88 mmol, 75.62% yield).

### (3) Synthesis of compound A-35

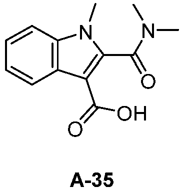

Compound **A-34** (430.00 mg, 1.88 mmol), sodium dihydrogen phosphate (381.26 mg, 2.44 mmol), hydrogen peroxide (76.70 mg, 2.26 mmol) and chlorous acid (238.00 mg, 2.63 mmol) were dissolved in acetonitrile/water (10 mL). The reaction mixture was reacted under stirring at room temperature overnight, concentrated and purified by medium-pressure liquid chromatography (MPLC) to give the compound **A-35** (300.00 mg, 1.22 mmol, 64.89% yield).

### (4) Synthesis of compound A19

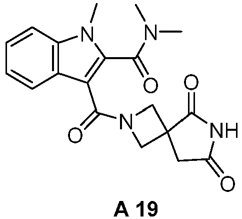

Compound **A-35** (8.54 mg, 60.91 µmol), compound **A-1** (15.00 mg, 60.91 µmol), EDCI (23.27 mg, 121.82 µmol) and pyridine (2 mL) were successively added to a 25 mL reaction flask. The reaction mixture was reacted under stirring in an ice bath for 0.5 h, quenched (monitored by LC-MS), and then added with a saturated NaCl solution (10 mL) and ethyl acetate (3 × 20 mL) for extraction. The organic phases were combined, dried over anhydrous sodium sulfate purified by medium-pressure liquid chromatography (MPLC) and concentrated under reduced pressure to give the compound **A-19** (5.50 mg, 14.93 µmol, 24.51% yield, 98.4% purity).

LC-MS: C₁₉H₂₁N₄O₄, [M+H]⁺ 369.2; found 369.2.

¹H NMR (600 MHz, DMSO-d₆) δ 11.19 (s, 1H), 7.69 (d, *J =* 8.4 Hz, 1H), 7.59 (d, *J =* 8.4 Hz, 1H), 7.31 (t, *J* = 7.8 Hz, 1H), 7.23 (t, *J =* 7.8 Hz, 1H), 4.25-3.94 (m, 4H), 3.71 (s, 3H), 3.08 (s, 3H), 2.97 (s, 2H), 2.91 (s, 3H).

### EXAMPLE 7 Preparation of compound A20

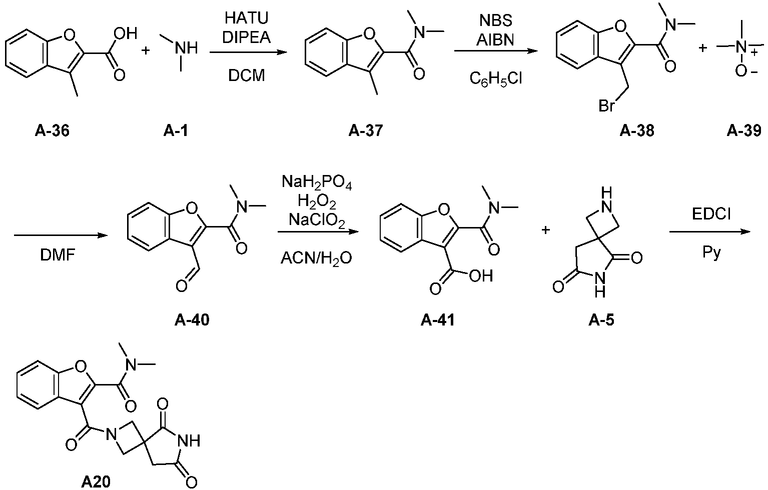

### (1) Synthesis of compound A-37

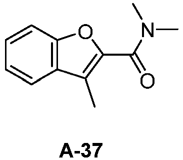

Compound **A-36** (410.00 mg, 2.33 mmol), HATU (1.06 g, 2.79 mmol), DIPEA (902.37 mg, 6.98 mmol, 1.22 mL) and dichloromethane (10 mL) were successively added to a 50 mL reaction flask. The reaction mixture was reacted under stirring in an ice bath for 20 min, followed by addition of the compound A-1 (128.67 mg, 2.85 mmol). Then the reaction mixture was reacted under stirring at room temperature for 1 h, concentrated and purified by medium-pressure liquid chromatography (MPLC) to give the compound **A-37** (397.00 mg, 1.95 mmol, 83.93% yield).

### (2) Synthesis of compound A-38

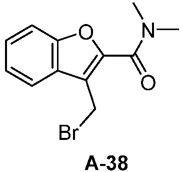

Compound A-37 (560.00 mg, 2.76 mmol), NBS (637.54 mg, 3.58 mmol), AIBN (22.62 mg, 137.77 µmol) and chlorobenzene (10 mL) were successively added to a 50 mL reaction flask. The reaction mixture was refluxed overnight, concentrated and purified by medium-pressure liquid chromatography (MPLC) to give the compound **A-38** (449.00 mg, 1.59 mmol, 57.76% yield).

### (3) Synthesis of compound A-40

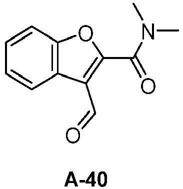

Compound **A-38** (449.00 mg, 1.59 mmol), compound **A-39** (478.13 mg, 6.37 mmol) and DMF (5 mL) were successively added to a 50 mL reaction flask. The reaction mixture was reacted under stirring overnight at room temperature, and purified by medium-pressure liquid chromatography (MPLC) to give the compound **A-40** (187.00 mg, 860.88 µmol, 54.09% yield).

### (4) Synthesis of compound A-41

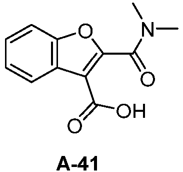

Compound **A-40** (187.00 mg, 860.88 µmol), sodium dihydrogen phosphate (381.26 mg, 2.44 mmol), hydrogen peroxide (35.14 mg, 1.03 mmol) and sodium chlorite (174.59 mg, 1.12 mmol) were dissolved in acetonitrile/water (10 mL). The reaction mixture was reacted under stirring overnight at room temperature, concentrated and purified by medium-pressure liquid chromatography (MPLC) to give the compound **A-41** (140.00 mg, 600.29 µmol, 69.73% yield).

### (5) Synthesis of compound A20

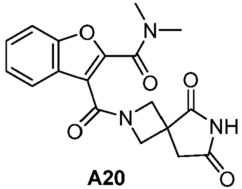

Compound **A-41** (30.00 mg, 128.63 µmol), compound **A-5** (18.03 mg, 128.63 µmol), EDCI (25.47 mg, 128.63 µmol) and pyridine (3 mL) were successively added to a 25 mL reaction flask. The reaction mixture was reacted under stirring in an ice bath for 0.5 h, quenched (monitored by LC-MS), and added with a saturated NaCl solution (10 mL) and ethyl acetate (3 × 20 mL) for extraction. The organic phases were combined, dried over anhydrous sodium sulfate, purified by medium-pressure liquid chromatography (MPLC) and concentrated under reduced pressure togive the compound **A-20** (8.00 mg, 22.51 µmol, 17.50% yield, 99.1% purity).

LC-MS: C₁₈H₁₈N₃O₅, [M+H]+ 355.1; found 355.2.

¹H NMR (400 MHz, DMSO-d₆) δ 11.23 (s, 1H), 7.78 (d, *J =* 7.6 Hz, 1H), 7.72 (d, *J =* 8.4 Hz, 1H), 7.50 (t, *J =* 7.6 Hz, 1H), 7.42 (t, *J =* 7.6 Hz, 1H), 4.35-3.93 (m, 4H), 3.07 (m, 6H) and 2.96 (m, 2H).

The technical effects of the present disclosure were illustrated through the following experiments.

### Experiment 1 Detection of compounds inhibition of CRBN/DDB1 protein activity (using a fluorescence resonance energy transfer (FRET) method)

### (1) Experimental materials and reagents

Microplate reader (BMG PHERAstar FSX), ECHO (LABCYTE Echo 665), microplate thermostatic oscillator (Hangzhou Ruicheng Instrument Co., Ltd.), disodium hydrogen phosphate (Sigma-Aldrich (Shanghai) Trading Co., Ltd.), sodium dihydrogen phosphate (Sigma-Aldrich (Shanghai) Trading Co., Ltd.), bovine serum albumin (Sigma-Aldrich (Shanghai) Trading Co., Ltd.), Anti-6His-Tb crypate Gold (Cisbio Bioassays Company CISBIO), CRBN/DDB1 protein (HitGen Inc.), 384-well plate (Grenier Bio-one (Shanghai) Co., Ltd.).

### (2) Experimental methods

The compound powders were dissolved in DMSO and serially diluted using ECHO. The resulting dilutions were individually added to 384-well reaction plates, ensuring that the final concentration of DMSO in the entire reaction system (10.0 µL) was 1.0%. An equal volume of DMSO was added as a control.

The CRBN/DDB1 protein was diluted to twice the required final concentration (5.0 nM) by addition of 20 mM disodium hydrogen phosphate, 20 mM sodium dihydrogen phosphate, 0.08% bovine serum albumin and a buffer with pH 7.0. The diluted CRBN/DDB1 protein (5.0 µL) was added to the 384-well reaction plates that already had the compounds added, centrifuged at 1000 rpm for 1 min, then pre-incubated in a microplate thermostatic oscillator at 25°C and 250 rpm for 15 min. The Anti-6His-Tb crypate Gold and FITC-labeled thalidomide analog were diluted to twice the required final concentration by addition of 20 mM disodium hydrogen phosphate, 20 mM sodium dihydrogen phosphate, 0.08% bovine serum albumin and the buffer with pH 7.0. The final concentration of Anti-6His-Tb crypate Gold was 0.2 nM, and the final concentration of the FITC-labeled thalidomide analog was 50.0 nM, so as to obtain a mixture of Anti-6His-Tb crypate Gold/FITC-labeled thalidomide analog. 5.0 µL of the mixture of Anti-6His-Tb crypate Gold/FITC-labeled thalidomide analog was individually added to the 384-well reaction plates, followed by centrifugation at 1000 rpm for 1 min. Then the reaction plates were incubated in the microplate thermostatic oscillator at 25°C and 250 rpm for 30 min. After the reaction was completed, the fluorescence signal values in the 384-well reaction plates were read using the microplate reader (Ex = 337 nm, Em = 520/490 nm).

### (3) Data analysis

A solvent group (containing 5.0 nM CRBN/DDB1, 0.2 nM Anti-6His-Tb crypate Gold, 50.0 nM FITC-labeled thalidomide analog and 1.0% DMSO) was used as a negative control. A reaction buffer group (containing 0.2 nM Anti-6His-Tb crypate Gold, 50.0 nM FITC-labeled thalidomide analog and 1.0% DMSO) was used as the blank control.

The residual activity percentage at each concentration was calculated using the following formula: residual activity (%) = 100% × (Flu compound group - Flu blank control) / (Flu negative control - Flu blank control).

A dose-effect curve was fitted using GraphPad 6.0 to calculate the IC₅₀ value.

**Table 1: Inhibition of CRBN/DDB1 protein by compounds**

| Compound number | IC₅₀ | Compound number | IC₅₀ | Compound number | IC₅₀ |
|---|---|---|---|---|---|
| A1 | +++++ | A8 | +++++ | A15 | +++ |
| A2 | ++++ | A9 | +++++ | A16 | +++ |
| A3 | +++++ | A10 | ++++ | A17 | + |
| A4 | +++ | A11 | +++++ | A18 | + |
| A5 | +++ | A12 | +++ | A19 | +++++ |
| A6 | ++++ | A13 | +++ | A20 | +++++ |
| A7 | +++++ | A14 | ++++ | | |

| | | | | | |
|---|---|---|---|---|---|
| "+" represented 200 µM > IC50 > 100 µM, "++" represented 100 µM > IC50 > 10 µM, "+++" represented 10 µM > IC50 > 1 µM, "++++" represented 1 µM > IC50 > 0.1 µM and "+++++" represented IC50 < 0.1 µM. | | | | | |

### Experiment 2 Isothermal titration calorimetry (ITC) detection of compound binding to CRBN/DDB 1

### (1) Experimental materials and reagents

Disodium hydrogen phosphate (Sigma-Aldrich (Shanghai) Trading Co., Ltd.), sodium dihydrogen phosphate (Sigma-Aldrich (Shanghai) Trading Co., Ltd.), Tween 20 (Sigma-Aldrich (Shanghai) Trading Co., Ltd.), dimethyl sulfoxide (Sigma-Aldrich (Shanghai) Trading Co., Ltd.), desalting column (Thermo Fisher Scientific Inc., #89882), microplate reader (BMG LABTECH Inc., PHERAstar FSX), CRBN/DDB1 protein (HitGen Inc.), MicroCal PEAQ-ITC (Malvern Panalytical Ltd.).

### (2) Experimental methods

6.1 mL of 200 mM disodium hydrogen phosphate aqueous solution and 3.9 mL of 200 mM sodium dihydrogen phosphate aqueous solution were mixed to obtain 200 mM PB buffer (pH 7.0). Then, 5397 µL of deionized water was mixed with 600 µL of 200 mM PB buffer and 3 µL of 10% Tween 20 aqueous solution to prepare a detection buffer with a pH of 7.0. The protein storage solution was subjected to buffer exchange using the detection buffer prepared above according to the instruction of the Zeba^{™} Spin Desalting Columns (Thermo Fisher Scientific Inc., #89882). After the exchange was completed, ultraviolet (UV) absorption of the protein solution at OD₂₈₀ₙₘ was measured using the microplate reader (BMG LABTECH Inc.,PHERAstar FSX), and the concentration was calculated. Based on the measured concentration, the protein was diluted to 10 µM with the detection buffer, and added with DMSO to a final concentration of 1%, resulting in a total volume of 300 µL. After a brief centrifugation at room temperature, the solution was collected for later use.

The compound powder was dissolved in DMSO and diluted to 100 µM with the detection buffer prepared above. The DMSO concentration was adjusted to a final concentration of 1% with a total volume of 100 µL. The compound solution was subjected to centrifugation at 15,000 rpm for 5 min at room temperature, and at least 75 µL of supernatant was collected for later use.

The ITC instrument (MicroCal PEAQ-ITC) was cleaned according to the instrument's procedures. After cleaning, a water-drop test was performed to check the cleanliness and status of the instrument. During the sample titration, the protein sample was added to a sample cell, and the compound solution was added to the titrator. The instrument temperature was set to 25°C, with a reference power (ucal/s) of 5.00, a feedback mode set to "High" and a stirring speed of 750 rpm. A total of 19 injections were added, with the first drop discarded. For the remaining 18 injections, 2 µL was added every 4 seconds, and an equilibration time of 150 seconds was allowed between each drop. After the titration was completed, the instrument was cleaned again. The water-drop test was performed again to ensure the cleanliness and proper status of the instrument. Then, a control experiment titration was carried out. The compound was prepared as described above and added to the titrator. Another 300 µL of detection buffer containing 1% DMSO was added to the sample cell. The instrument parameters were set the same as above and the titration was performed again.

### (3) Data analysis

The fitting was performed with a "one set of sites" model using the self-analysis software of the ITC instrument. After background subtraction from the control experiment, reaction-related parameters such as N, KD, δH, δG and δS were obtained.

**Table 2: Compound and CRBN/DDB 1 protein binding results**

| Compound number | KD_{ITC} | Compound number | KD_{ITC} |
|---|---|---|---|
| A1 | 0.020 µM | A3 | 0.015 µM |

The above experiments demonstrated that the compound A of the embodiments of the present disclosure exhibited good CRBN binding affinity and inhibitory effects, and could be effectively used for the treatment of disease associated with abnormal CRBN activity.

Described above are merely preferred embodiments of the present disclosure, and are not intended to limit the scope of the present disclosure. It should be understood that various modifications, changes and replacements made by those skilled in the art without departing from the spirit of the disclosure shall fall within the scope of the present disclosure defined by the appended claims.

## Claims

1. A compound of formula (I), or a stereoisomer, a deuterated compound or a pharmaceutically acceptable salt thereof: 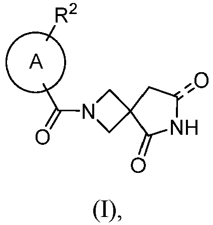 **characterized in that**:
==∘ represents presence or absence of oxygen substitution;
A ring is selected from the group consisting of 3 to 12-membered cycloalkyl, 4 to 12-membered heterocycloalkyl, 6 to 10-membered aromatic ring, and 5 to 10-membered heteroaromatic ring, wherein cycloalkyl, heterocycloalkyl, aromatic ring and heteroaromatic ring are independently unsubstituted or substituted with one, two, three or four R^{A1};
each R^{A1} is independently selected from the group consisting of hydrogen, halogen, cyano group, nitro, =O, =S, =CR^{A2}R^{A3}, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR^{A2}, -C₀₋₄ alkylidene-OC(O)R^{A2}, -C₀₋₄ alkylidene-SR^{A2}, -C₀₋₄ alkylidene-S(O)₂R^{A2}, -C₀₋₄ alkylidene-S(O)R^{A2}, -C₀₋₄ alkylidene-S(O)₂NR^{A2}R^{A3}, -C₀₋₄ alkylidene-S(O)NR^{A2}R^{A3}, -C₀₋₄ alkylidene-C(O)R^{A2}, -C₀₋₄ alkylidene-C(O)OR^{A2}, -C₀₋₄ alkylidene-C(O)NR^{A2}R^{A3}, -C₀₋₄ alkylidene-NR^{A2}R^{A3}, -C₀₋₄ alkylidene-NR^{A2}C(O)R^{A-3}, -C₀₋₄ alkylidene-NR^{A2}S(O)₂R^{A3}, -C₀₋₄ alkylidene-NR^{A2}S(O)R^{A3}, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R^{A4};
each R^{A4} is independently selected from the group consisting of hydrogen, halogen, cyano group, nitro, =O, =S, =CR^{A2}R^{A3}, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR^{A2}, -C₀₋₄ alkylidene-OC(O)R^{A2}, -C₀₋₄ alkylidene-SR^{A2}, -C₀₋₄ alkylidene-S(O)₂R^{A2}, -C₀₋₄ alkylidene-S(O)R^{A2}, -C₀₋₄ alkylidene-S(O)₂NR^{A2}R^{A3}, -C₀₋₄ alkylidene-S(O)NR^{A2}R^{A3}, -C₀₋₄ alkylidene-C(O)R^{A2}, -C₀₋₄ alkylidene-C(O)OR^{A2}, -C₀₋₄ alkylidene-C(O)NR^{A2}R^{A3}, -C₀₋₄ alkylidene-NR^{A2}R^{A3}, -C₀₋₄ alkylidene-NR^{A2}C(O)R^{A-3}, -C₀₋₄ alkylidene-NR^{A2}S(O)₂R^{A-3} and -C₀₋₄ alkylidene-NR^{A2}S(O)R^{A3};
R^{A2} and R^{A3} are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl;
R² is selected from the group consisting of hydrogen, halogen, cyano group, nitro, =O, =S, =CR²¹R²², -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-SR²¹, -C₀₋₄ alkylidene-S(O)₂R²¹, -C₀₋₄ alkylidene-S(O)R²¹, -C₀₋₄ alkylidene-S(O)₂NR²¹R²², -C₀₋₄ alkylidene-S(O)NR²¹R²², -C₀₋₄ alkylidene-C(O)R²¹, -C₀₋₄ alkylidene-C(O)OR²¹, -C₀₋₄ alkylidene-C(O)NR²¹R²², -C₀₋₄ alkylidene-NR²¹R²², -C₀₋₄ alkylidene-NR²¹C(O)R²², -C₀₋₄ alkylidene-NR²¹S(O)₂R²², -C₀₋₄ alkylidene-NR²¹S(O)R²², -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²³;
each R²³ is independently selected from the group consisting of hydrogen, halogen, cyano group, nitro, =O, =S, =CR²¹R²², -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-SR²¹, -C₀₋₄ alkylidene-S(O)₂R²¹, -C₀₋₄ alkylidene-S(O)R²¹, -C₀₋₄ alkylidene-S(O)₂NR²¹R²², -C₀₋₄ alkylidene-S(O)NR²¹R²², -C₀₋₄ alkylidene-C(O)R²¹, -C₀₋₄ alkylidene-C(O)OR²¹, -C₀₋₄ alkylidene-C(O)NR²¹R²², -C₀₋₄ alkylidene-NR²¹R²², -C₀₋₄ alkylidene-NR²¹C(O)R²², -C₀₋₄ alkylidene-NR²¹S(O)₂R²², -C₀₋₄ alkylidene-NR²¹S(O)R²², -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
R²¹ and R²² are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₁₋₄ alkylidene-OR²⁴, -C₁₋₄ alkylidene-OC(O)R²⁴, -C₁₋₄ alkylidene-SR²⁴, -C₁₋₄ alkylidene-S(O)₂R²⁴, -C₁₋₄ alkylidene-S(O)R²⁴, -C₁₋₄ alkylidene-S(O)₂NR²⁴R²⁵, -C₁₋₄ alkylidene-S(O)NR²⁴R²⁵, -C₁₋₄ alkylidene-C(O)R²⁴, -C₁₋₄ alkylidene-C(O)OR²⁴, -C₁₋₄ alkylidene-C(O)NR²⁴R²⁵, -C₁₋₄ alkylidene-NR²⁴R²⁵, -C₁₋₄ alkylidene-NR²⁴C(O)R²⁵, -C₁₋₄ alkylidene-NR²⁴S(O)₂R²⁵, -C₁₋₄ alkylidene-NR²⁴S(O)R²⁵, - C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
each R²⁶ is independently selected from the group consisting of hydrogen, halogen, cyano group, nitro, =O, =S, =CR²⁴R²⁵, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²⁴, -C₀₋₄ alkylidene-OC(O)R²⁴, -C₀₋₄ alkylidene-SR²⁴, -C₀₋₄ alkylidene-S(O)₂R²⁴, -C₀₋₄ alkylidene-S(O)R²⁴, -C₀₋₄ alkylidene-S(O)₂NR²⁴R²⁵, -C₀₋₄ alkylidene-S(O)NR²⁴R²⁵, -C₀₋₄ alkylidene-C(O)R²⁴, -C₀₋₄ alkylidene-C(O)OR²⁴, -C₀₋₄ alkylidene-C(O)NR²⁴R²⁵, -C₀₋₄ alkylidene-NR²⁴R²⁵, -C₀₋₄ alkylidene-NR²⁴C(O)R²⁵, -C₀₋₄ alkylidene-NR²⁴S(O)₂R²⁵, -C₀₋₄ alkylidene-NR²⁴S(O)R²⁵, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁷;
R²⁴ and R²⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl; and
each R²⁷ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl.

2. The compound of claim 1, **characterized in that** the A ring is selected from the group consisting of: 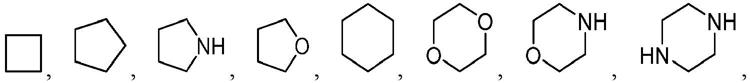 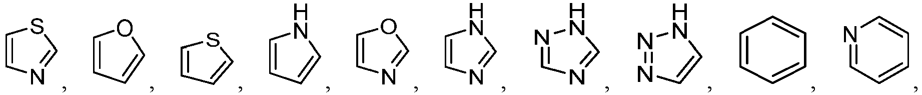 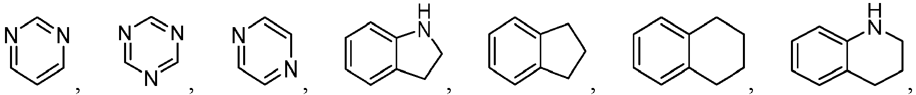 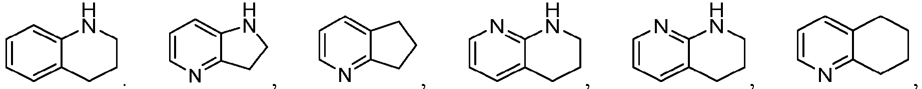 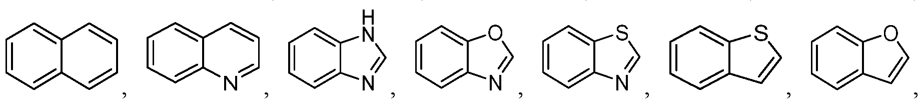 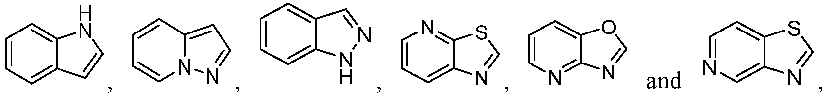 wherein the A ring is unsubstituted or substituted with one, two, three or four R^{A1}.

3. The compound of claim 2, **characterized in that** each R^{A1} is independently selected from the group consisting of hydrogen, halogen, cyano group, nitro, =O, =S, -C₁₋₃ alkyl and halogen-substituted -C₁₋₃ alkyl.

4. The compound of claim 1, **characterized in that** the A ring is selected from the group consisting of:

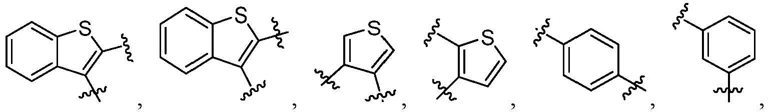

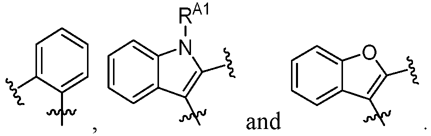

5. The compound of claim 1 or 4, **characterized in that** R² is selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-C(O)R²¹, -C₀₋₄ alkylidene-C(O)OR²¹, -C₀₋₄ alkylidene-C(O)NR²¹R²², -C₀₋₄ alkylidene-NR²¹R²², -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and - C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²³;
each R²³ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²¹, -C₀₋₄ alkylidene-OC(O)R²¹, -C₀₋₄ alkylidene-C(O)R²¹, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
R²¹ and R²² are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₁₋₄ alkylidene-OR²⁴, -C₁₋₄ alkylidene-OC(O)R²⁴, -C₁₋₄ alkylidene-C(O)R²⁴, -C₁₋₄ alkylidene-C(O)OR²⁴, -C₁₋₄ alkylidene-C(O)NR²⁴R²⁵, -C₁₋₄ alkylidene-NR²⁴R²⁵, -C₁₋₄ alkylidene-NR²⁴C(O)R²⁵, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring), wherein alkylidene, cycloalkyl, heterocycloalkyl, aromatic ring and heteroaromatic ring are independently unsubstituted or substituted with one, two, three or four R²⁶;
each R²⁶ is independently selected from the group consisting of hydrogen, halogen, cyano group, nitro, =O, =S, =CR²⁴R²⁵, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylidene-OR²⁴, -C₀₋₄ alkylidene-OC(O)R²⁴, -C₀₋₄ alkylidene-C(O)R²⁴, -C₀₋₄ alkylidene-C(O)OR²⁴, -C₀₋₄ alkylidene-C(O)NR²⁴R²⁵, -C₀₋₄ alkylidene-NR²⁴R²⁵, -C₀₋₄ alkylidene-NR²⁴C(O)R²⁵, -C₀₋₄ alkylidene-(3 to 10-membered cycloalkyl), -C₀₋₄ alkylidene-(4 to 10-membered heterocycloalkyl), -C₀₋₄ alkylidene-(6 to 10-membered aromatic ring) and -C₀₋₄ alkylidene-(5 to 10-membered heteroaromatic ring); and
R²⁴ and R²⁵ are each independently selected from the group consisting of hydrogen, - C₁₋₃ alkyl and halogen-substituted -C₁₋₃ alkyl.

6. The compound of claim 5, **characterized in that** R² is selected from the group consisting of -C(O)NR²¹R²², -C(O)R²¹, -C_{0~2} alkylidene-NR²¹R²² and -C(O)OR²¹;
R²¹ and R²² are each independently selected from the group consisting of hydrogen, - C₁₋₃ alkyl, -C₀₋₁ alkylidene-(6-membered aromatic ring), -C₀₋₁ alkylidene-(10-membered heteroaromatic ring), -(4 to 6-membered heterocycloalkyl) and -(3 to 6-membered cycloalkyl), wherein aromatic ring, heteroaromatic ring, heterocycloalkyl and cycloalkyl are independently unsubstituted or substituted with one, two, three or four R²⁶;
each R²⁶ is independently selected from the group consisting of hydrogen, -C₁₋₃ alkyl, -(4 to 6-membered heterocycloalkyl), -C(O)R²⁴, -C(O)OR²⁴ and -OC(O)R²⁴; and
R²⁴ is selected from the group consisting of hydrogen, methyl and ethyl.

7. The compound of claim 6, **characterized in that** R² is selected from the group consisting of: hydrogen,

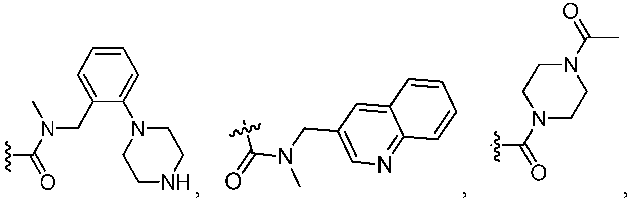

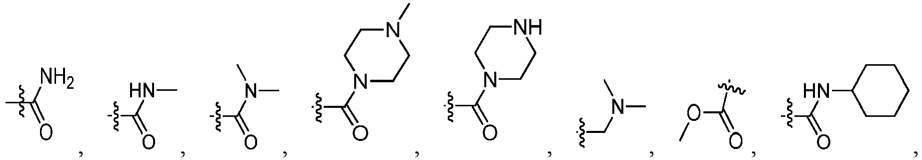

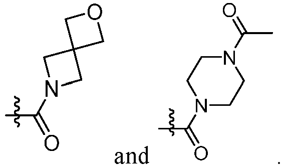

8. The compound of any one of claims 1-7, **characterized in that** the compound is selected from the group consisting of:

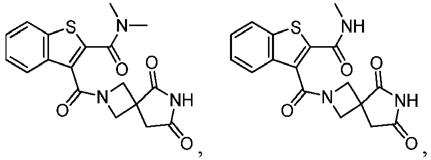

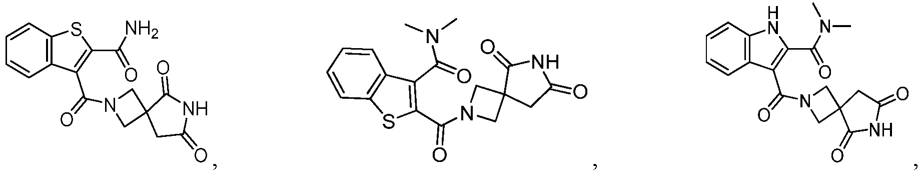

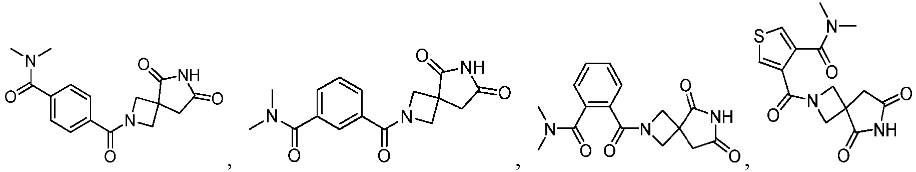

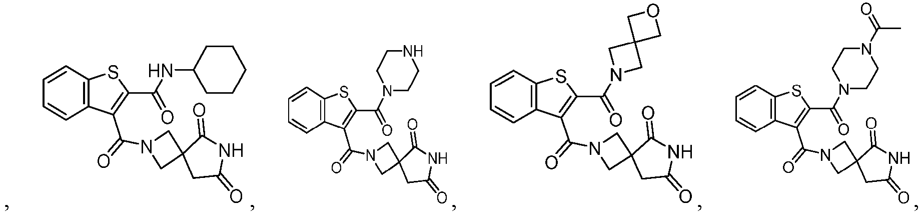

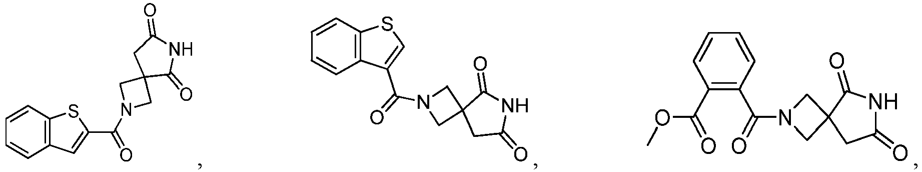

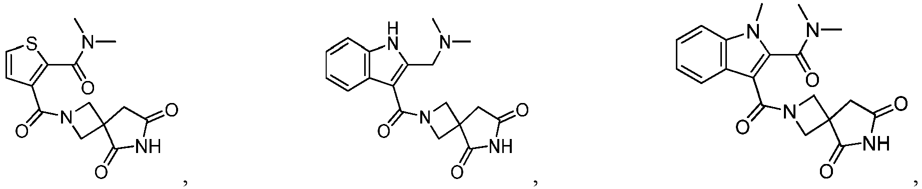

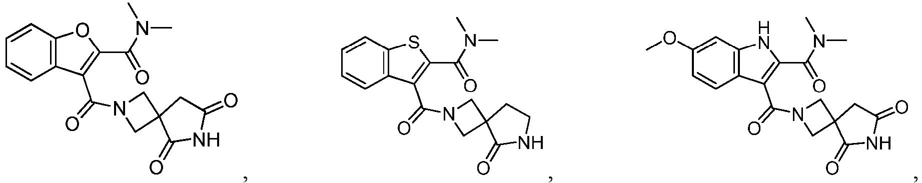

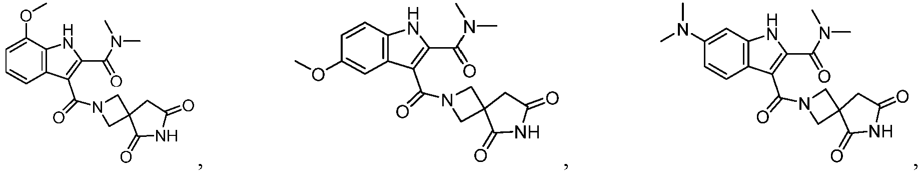

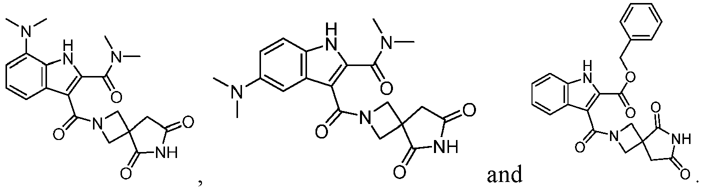

9. The compound of any one of claims 1-8, or a stereoisomer, a deuterated compound or a pharmaceutically acceptable salt thereof for use in treatment of a disease associated with abnormal cell proliferation.

10. The compound for use of claim 9, **characterized in that** the disease is a cancer.

11. The compound of any one of claims 1-8, or a stereoisomer, a deuterated compound or a pharmaceutically acceptable salt thereof for use in preparation of a drug for targeted protein degradation.

12. The compound of any one of claims 1-8, or a stereoisomer, a deuterated compound or a pharmaceutically acceptable salt thereof for use as an intermediate in preparation of a drug for targeted protein degradation.
